# EUROPEAN PATENT SPECIFICATION

(11) **EP 4 442 214 B1**
(45) Date of publication and mention of the grant of the patent: **04.02.2026**
(21) Application number: 24196199.4
(22) Date of filing: 30.03.2021
(51) Int. Cl.: A61B 17/072, A61B 50/30, A61B 17/00, A61B 17/32

(54) **SEPARATION MEMBER FOR SURGICAL INSTRUMENT AND SURGICAL INSTRUMENT**
TRENNELEMENT FÜR CHIRURGISCHES INSTRUMENT UND CHIRURGISCHES INSTRUMENT
ÉLÉMENT DE SÉPARATION POUR INSTRUMENT CHIRURGICAL ET INSTRUMENT CHIRURGICAL

(30) Priority: 03.04.2020 CN 202010261072
(43) Date of publication of application: 09.10.2024
(62) Divisional of application: 21780517.5
(73) Proprietor: Fulbright Medical Inc., Wuxi, Jiangsu 214437 (CN)
(72) Inventor: SUN, Baofeng, Wuxi, Jiangsu, 214437 (CN); FU, Jianxin, Wuxi, Jiangsu, 214437 (CN); ZHOU, Xiaopeng, Wuxi, Jiangsu, 214437 (CN)
(74) Representative: Maiwald GmbH

(56) References cited:
- US-A1- 2012 138 661
- US-A1- 2013 317 305
- US-A1- 2016 317 759
- US-A1- 2017 367 729
- US-A1- 2019 059 896

## Description

### Cross-Reference to Related Application

The present invention is a divisional application of the European patent application with the application number 21780517.5.

### Technical Field

The present invention relates to the technical field of medical apparatus and instruments, and in particular, to a separation member for a surgical instrument

The present invention further relates to a surgical instrument. The surgical instrument includes the above separation member.

### Background

A stapler suitable for surgery is a surgical instrument that can excise redundant tissue while suturing a wound of a patient, and is widely applied to resection and anastomosis of tissue in minimally invasive surgery such as abdominal surgery, gynecology departments, pediatric departments and thoracic surgery. The stapler enters a human body by using a cannula of a puncture outfit that accurately positions a surgical site, and then makes a longitudinal incision in the tissue and applies a suturing staple on an opposite side of the incision, so as to dissect and anastomose the tissue, which is similar to a book sewer. The stapler includes an end effector. The end effector includes a staple cartridge base and a staple abutting seat. The staple cartridge base is used to receive a staple cartridge. The stapler further includes a cutting component. The cutting component is operationally supported relative to the staple cartridge base. Once a doctor determines that the end effector is appropriately grasp the tissue and in a closed position, the stapler can be started to cut and suture the tissue.

During surgery, the doctor sometimes needs to cut and suture specific target tissue, but the target tissue is attached to other tissue. For example, the doctor sometimes needs to cut and suture a blood vessel, but the blood vessel is usually attached to other tissue. In order to cut and suture the blood vessel, the doctor needs to strip the blood vessel from other tissue, then makes the blood vessel be between the staple abutting seat and the staple cartridge base, and finally uses the stapler to cut and suture the blood vessel.

When the doctor operates a conventional surgical instrument, and if the doctor needs to cut and suture the specific target tissue, the doctor usually needs to repeatedly adjust an angle of the surgical instrument to cause a far end of the end effector to align the target tissue, applies force to the surgical instrument in a direction toward the far end, to cause the far end of the end effector to separate the target tissue and then to cause the target tissue to be between the staple abutting seat and the staple cartridge base, so as to cut and suture the target tissue.

However, the design of the far end of the conventional end effector is not suitable for the stripping of the target tissue, and it is laborious for the doctor to use the end effector of the surgical instrument to separate the target tissue.

US20120138661A1 discloses a surgical fastener applying apparatus that includes an elongate body portion having proximal and distal ends, an end effector including a first movably coupled to a second jaw that is positioned at the distal end of the elongate body portion, and an introducer member. The introducer member has proximal and distal portions, and is configured and dimensioned for releasable connection with the end effector. The introducer member is at least partially formed from a flexible material, and is configured and dimensioned to separate target tissue from collateral tissue prior to positioning of the target tissue between the first and second jaws of the end effector.

US20190059896A1 discloses a surgical stapling apparatus. The surgical stapling apparatus includes a handle assembly, an elongate tubular body, a loading unit including an anvil assembly and a staple cartridge assembly, and a reload assembly. The reload assembly includes a staple cartridge releasably disposed within the staple cartridge assembly of the loading unit, a cartridge buttress releasably secured to the staple cartridge, and an anvil buttress including a proximal portion releasably secured to the staple cartridge and a distal portion releasably coupled to the anvil assembly of the loading unit.

US20170367729A1 discloses apparatuses, systems, kits, and methods for treating skin. Such as skin tightening or for treating diseases, disorders, and conditions that would benefit from tissue area or volume reduction, skin restoration, skin tightening, skin lifting, and/or skin repositioning and/or for generally improving skin function or appearance. Such apparatuses, systems, kits, and methods include an apparatus having a handheld main body and a detachably attachable tip including one or more needles.

### Summary

The invention is set out in the appended set of claims.

In view of the deficiency in the prior art, the present invention is intended to provide a separation member for a surgical instrument and the surgical instrument.

In an embodiment mode, the separation member includes a mounting portion and a working portion, the mounting portion is connected to the working portion, the mounting portion includes a clipping portion, the working portion is configured to strip a tissue, the clipping portion is in elastically-mated connection with or detached from a surgical instrument, so that the mounting portion is able to be detachably connected to the surgical instrument.

In an embodiment mode, in a first state, the clipping portion is clipped in a clipping port of the surgical instrument under an action of elastic force, so that the separation member is in elastically-mated connection with the surgical instrument; in a second state, the clipping portion overcomes the elastic force to disengage from the clipping port, so that the separation member is detached from the surgical instrument.

In an embodiment mode, the mounting portion includes an insertion portion, the clipping portion has a first end and a second end, the first end is connected to the insertion portion, the second end is in elastically-mated connection with or detached from the surgical instrument, the insertion portion and the clipping portion are configured to insert into an insertion channel of the surgical instrument.

In an embodiment mode, the second end rotates around the first end, the first end includes a rotating shaft, the insertion portion is provided with a shaft hole, the rotating shaft is mounted in the shaft hole, the rotating shaft rotates in the shaft hole to cause the clipping portion to rotate.

In an embodiment mode, the first end is mounted to a first side surface of the insertion portion, a second side surface of the insertion portion is located on an opposite side of the first side surface, the insertion portion is provided with an opening through the first side surface and the second side surface, so that the second end protrudes from the first side surface by extending through the opening from the second side surface.

In an embodiment mode, the first side surface is recessed inward to form accommodating space, the accommodating space includes the opening penetrating the insertion portion, the second end protrudes from the opening to be in clipped connection with the clipping port in the first state.

In an embodiment mode, the separation member includes a support portion, the support portion elastically supports the clipping portion, when the separation member is in the first state, a part of the clipping portion is accommodated in the accommodating space, a part of the support portion is also accommodated in the accommodating space.

In an embodiment mode, the separation member includes a support portion, the support portion elastically supports the clipping portion, the support portion has a first portion and a second portion, the insertion portion has a first side surface and a second side surface arranged opposite to each other, the first portion is connected to the first side surface, the second portion abuts against the clipping portion and provides the elastic force for the clipping portion,

In an embodiment mode, the second end rotates around the first end, the first end includes a rotating shaft, the insertion portion is provided with a shaft hole, the shaft hole is partially open, a part of the rotating shaft is arranged on the shaft hole, the support portion covers an open portion of the shaft hole.

In an embodiment mode, the separation member includes a second elastomer, the second elastomer is arranged between the clipping portion and the support portion, the second elastomer and the support portion provide the elastic force for the clipping portion together.

In an embodiment mode, the second end includes a clipping surface and a slope disposed with the clipping surface at an angle, the clipping surface extends vertically, the clipping surface abuts against a sidewall of the clipping port to prevent the separation member from falling off the surgical instrument, the slope is configured to guide the second end into the clipping port.

In an embodiment mode, a second wedge portion is disposed on a near end of the working portion, the second wedge portion has a second slope, the separation member is mounted to the staple abutting seat, a first slope of the first wedge portion at a far end of the staple abutting seat abuts against the second slope.

In an embodiment mode, the separation member has an elastic member, the elastic member is arranged on a surface of the working portion.

In an embodiment mode, the separation member is provided with a slot, the elastic member is disposed in the slot, an outside surface of the elastic member is protrudes from a second outer surface of the separation member or is flush with the second outer surface.

In an embodiment mode, the elastic member is in a bag shape, at least of the working portion is inserted in the elastic member.

In an embodiment mode, the second end rotates around the first end, the first end includes a rotating shaft, the first end of the clipping portion is provided with a pin hole extending through the rotating shaft, the mounting portion of the separation member is provided with circular holes corresponding to two end portions of the pin hole, the rotating shaft extends through a first circular hole of the circular holes, the pin hole and a second circular hole of the circular holes, so that the first end of the clipping portion is rotatably mounted on the mounting portion.

In an embodiment mode, the clipping portion is at least partially made of the elastic material, a near end of the clipping portion is connected to the mounting portion, the clipping portion is deformed, a far end of the clipping portion rotates around the near end, so that the clipping portion is clipped in a clipping port or detached from a clipping port of the surgical instrument.

In an embodiment mode, the clipping portion is at least partially made of the elastic material, a far end of the clipping portion is fixed to a portion of the separation member other than the clipping portion, the clipping portion is deformed, so that a near end of the clipping portion is in mated connection with or detached from a clipping port of the surgical instrument.

In an embodiment mode, the separation member is provided with an accommodation cavity, the accommodation cavity extends from a near end of the mounting portion in a direction toward the working portion, the accommodation cavity is partially located in the working portion, a mounting shaft vertically extending is disposed on a far end of the accommodation cavity, the clipping portion is provided with a mounting hole that is able to be sleeved on the mounting shaft, a rotation axis of the clipping portion is parallel to a working surface of the separation member.

In an embodiment mode, the surgical instrument includes an end effector, the end effector is connected to the separation member, the separation member is detachably connected to the end effector of the surgical instrument.

Therefore, the mounting portion of the separation member is detachably connected to the surgical instrument through insertion and clipping, thereby achieving reliable mounting and convenient dismounting.

### Brief Description of the Drawings

Fig. 1 is a front view of a surgical instrument according to the present invention.
Fig. 2 is a perspective matching view of a staple abutting seat of the surgical instrument shown in Fig. 1 and a separation member, showing a front side and partial back side, respectively.
Fig. 3 and Fig. 4 are three-dimensional views of the separation member shown in Fig. 2.
Fig. 5 is a three-dimensional exploded view of the separation member shown in Fig. 4.
Fig. 6 is a partial three-dimensional view of a staple abutting seat of the surgical instrument shown in Fig. 1.
Fig. 7 and Fig. 8 are three-dimensional views of a combination formed by a staple abutting seat, a separation member and a packaging box.
Fig. 9 and Fig. 10 are three-dimensional exploded views of the combination shown in Fig. 8.
Fig. 11 is a three-dimensional view of a packaging box shown in Fig. 10.
Fig. 12 and Fig. 13 are cross-sectional views in a longitudinal direction in Fig. 7; and state changes of Fig. 12 to Fig. 13 show a state changing process of using a packaging box to detach a separation member.
Fig. 14 is an enlarged view of a part A in Fig. 12.
Fig. 15 is an enlarged view of a part B in Fig. 12.
Fig. 16 is a three-dimensional view of a separation member according to a second implementation of the present invention. Fig. 17 is a three-dimensional exploded view of the separation member shown in Fig. 16.
Fig. 18 is a three-dimensional exploded view of a separation member according to a third implementation of the present invention.
Fig. 19 is a three-dimensional view of a separation member according to a fourth implementation of the present invention.
Fig. 20 is a three-dimensional exploded view of the separation member shown in Fig. 19.
Fig. 21 is a three-dimensional view of a separation member mounted to a staple abutting seat according to a fifth implementation of the present invention.
Fig. 22 is a three-dimensional view of the separation member shown in Fig. 21.
Fig. 23 is a three-dimensional view of a separation member mounted to a staple abutting seat according to a sixth implementation of the present invention.
Fig. 24 and Fig. 25 are three-dimensional exploded views of Fig. 23.
Fig. 26 is a three-dimensional view of the separation member shown in Fig. 23.
Fig. 27 is a three-dimensional view of a separation member mounted to a staple abutting seat according to a seventh implementation of the present invention.
Fig. 28 is a three-dimensional view of the separation member shown in Fig. 27.
Fig. 29 is a three-dimensional exploded view of the separation member shown in Fig. 28.

### Detailed Description of the Embodiments

To make the objectives, technical solutions and advantages of the present invention clearer, the present invention is further described in detail with reference to the drawings and embodiments. It should be understood that the specific examples described here are merely used to explain the present invention, and are not used to limit the present invention. Based on the embodiments in the present invention, all other embodiments obtained by those of ordinary skilled in the art without creative work shall fall within the protection scope of the present invention.

The terms "near", "rear", "far" and "front" are used herein with respect to a clinician manipulating a handle assembly of a surgical instrument. The terms "near" and "rear" refer to portions close to the clinician; and the terms "far" and "front" refer to portions away from the clinician. For example, the handle assembly is on a near side and a rear side, and an end effector is on a far side and a front side. For another example, a near side end of a certain part represents an end relatively close to the handle assembly, and a far side end represents an end relatively close to the end effector. In the present invention, a relative positional relationship between the parts of the separation member, for example, being relatively located on a "near" end or a "far" end, is based on the state in which the separation member is mounted to the surgical instrument. The terms "upper" and "lower" are based on relative positions of a staple abutting seat and a staple cartridge base of the end effector. Specifically, the staple abutting seat is at the "upper" portion, and the staple cartridge base is at the "lower" portion.

The "longitudinal direction" is defined as a direction from far to near or a direction from near to far. The "longitudinal direction" of the separation member is defined according to the state in which the separation member is mounted to the surgical instrument. The "longitudinal direction" of the packaging box is defined according to the state in which the separation member is mounted to the packaging box, that is, the longitudinal direction of the packaging box is parallel to the longitudinal direction of the separation member. In addition, that a component extends lengthwise means that a length of the component is greater than a width of the component, for example, the entire component is long-strip-shaped. However, it is to be understood that, "lengthwise extending" does not limit the specific shape and setting position of the component.

A surface of the separation member close to a side of target tissue is defined as a "front surface", and a surface away from the side of the target tissue is defined as a "back surface". Specifically, when the separation member is operated, a concave surface of the working portion is close to the target tissue and can strip the target tissue from other tissues. The surface of the side where the concave surface is located is defined as the front surface, and the surface of the side where a convex surface is located is defined as the back surface.

It is to be understood that, the orientations "near", "far", "rear", "front", "upper", "lower", "front surface", "back surface" are defined for ease of description. However, the surgical instrument may be used in various directions and positions. Therefore, these terms expressing relative positional relationships are not restrictive and absolute. The "longitudinal direction" is also defined for ease of description. In the present invention, the above definitions shall comply with the above explicit stipulations and limitations if there are other explicit stipulations and definitions.

In the present invention, unless expressly specified and limited otherwise, the terms such as "connected to each other" should be understood in a broad sense. For example, "connected to each other" may be a fixed connection, a detachable connection, a movable connection, or an integrated; or may be a direct connection or an indirect connection by means of an intermediate medium; or may be an internal communication between two elements or an interaction relationship between two elements. Except that "connection" does not include integration, "connection" has the same meaning as "connected to each other". For those of ordinary skill in the art, specific meanings of the above terms in the present invention may be understood according to a specific condition.

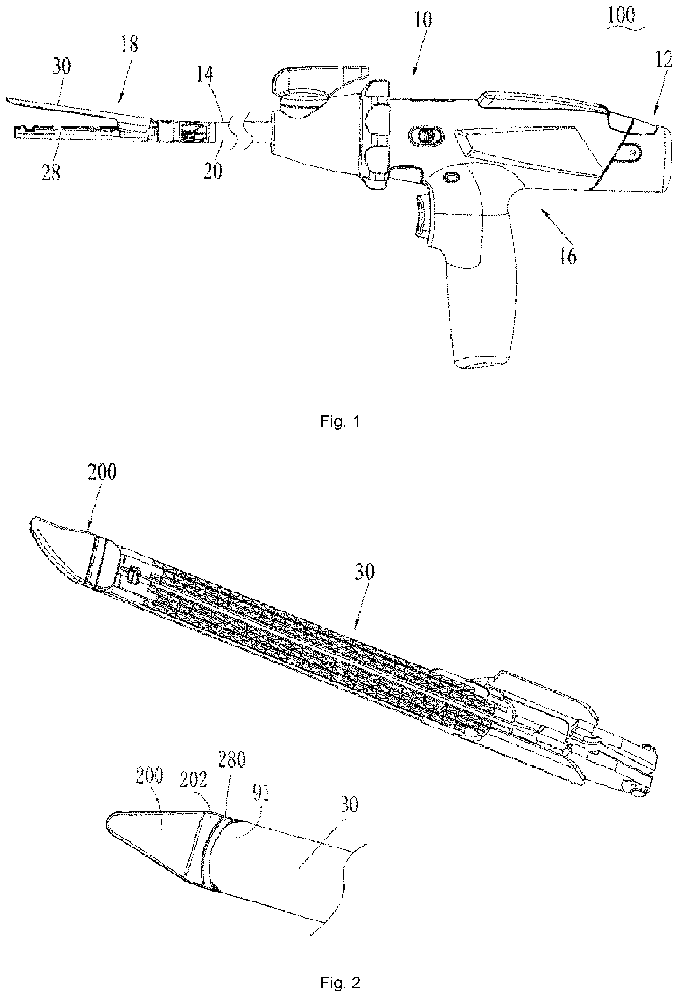

Fig. 1 shows a surgical instrument 100 in the present invention.

Referring to Fig. 1, the surgical instrument provided in the present invention is a stapler, preferably, an electric stapler. The surgical instrument 100 includes a host 10 and a battery pack 12 detachably mounted with the host 10. Those skilled in the art may think that, the surgical instrument may also be a manual stapler, and any solution that is the same as or similar to this implementation is covered within the protection scope of the present invention.

The host 10 includes a rod body assembly 14, a housing 16 disposed on one end of the rod body assembly 14, and an end effector 18 disposed on the other end of the rod body assembly 14. A cutting component (not shown) is disposed in the end effector 18.

The rod body assembly 14 includes a cannula 20 and a core shaft (not shown) accommodated in the cannula 20. A motor and a transmission mechanism are disposed in the housing 16. The motor drives the end effector 18 and the cutting component by using the transmission mechanism. The end effector 18 includes a staple cartridge base 28 and a staple abutting seat 30 rotatably connected to the staple cartridge base 28. The staple cartridge base 28 is used to operationally support a staple cartridge assembly (not shown) located therein. The staple abutting seat 30 may be selectively moved between an open position and a closed position, so as to clamp the tissue or loosen the tissue. The cutting component is disposed in the end effector 18 to cut the tissue. The staple cartridge assembly is used to suture the tissue.

The transmission mechanism includes an end effector driving apparatus. The end effector driving apparatus drives the staple abutting seat 30 of the end effector 18 to rotate relative to the staple cartridge base 28, so as to be selectively moved between the open position and the closed position. Generally, the end effector 18 driving apparatus includes a gear, a cam and the cannula 20. The gear drives the cannula 20 to do linear movement by means of the switching of the cam. By means of the linear movement of the cannula 20, the staple abutting seat 30 is driven to rotate by using a switching mechanism. A specific structure of the end effector driving apparatus is the same as or similar to that in the prior art, which is not described herein again.

The transmission mechanism further includes a cutting component driving apparatus. Generally, the cutting component driving apparatus includes a gear, a rack and a core shaft. The gear rotates to drive the rack, the core shaft and the cutting component to do linear movement, so that the cutting component may cut the tissue. A specific structure of the cutting component driving apparatus is the same as or similar to that in the prior art, which is not described herein again.

When the surgical instrument is used, the end effector driving apparatus first drives the cannula 20 to drive the staple abutting seat 30 to rotate to be mated with the staple cartridge base 28, so as to clamp the target tissue. Then, the motor starts and rotates forward; the cutting component driving apparatus drives the cutting component to advance by using the core shaft; and the cutting component moves from an initial position to a termination position in the end effector 18 of the surgical instrument, and synchronously pushes a staple-pushing block in the staple cartridge to move from the initial position to the termination position, so as to achieve an effect of suturing the cut tissue while redundant tissue is cut. Next, the motor rotates backward, and the cutting component driving apparatus drives the cutting component to retract to the initial position by using the core shaft. Then, the end effector driving apparatus drives the cannula 20 to drive the staple abutting seat 30 to open, so as to loosen the tissue. Finally, the end effector driving apparatus drives the cannula 20 to drive the staple abutting seat 30 to close. Definitely, before and after the surgical instrument is used, the staple abutting seat 30 is required to be closed relative to the staple cartridge base 28, so that the end effector 18 extends into a body of a patient or is removed from the body of the patient via the cannula of a puncture outfit, and details are not described again.

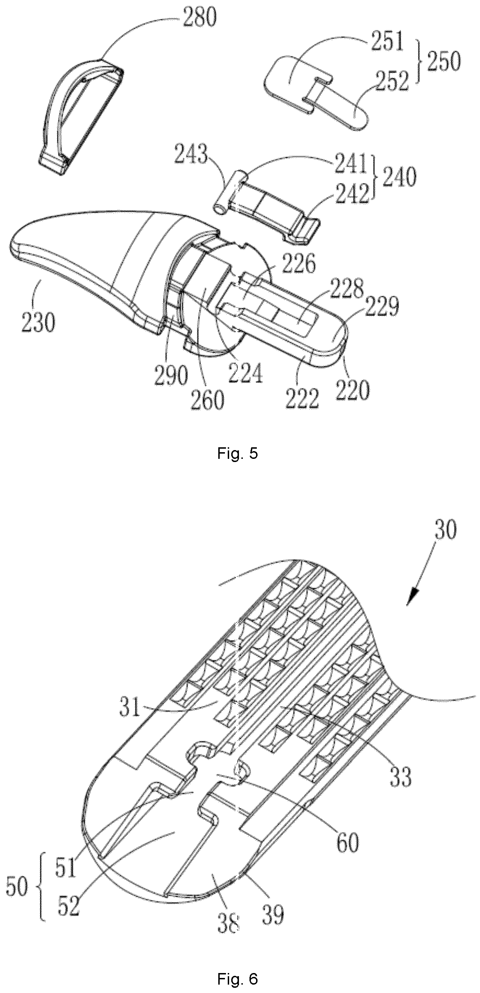

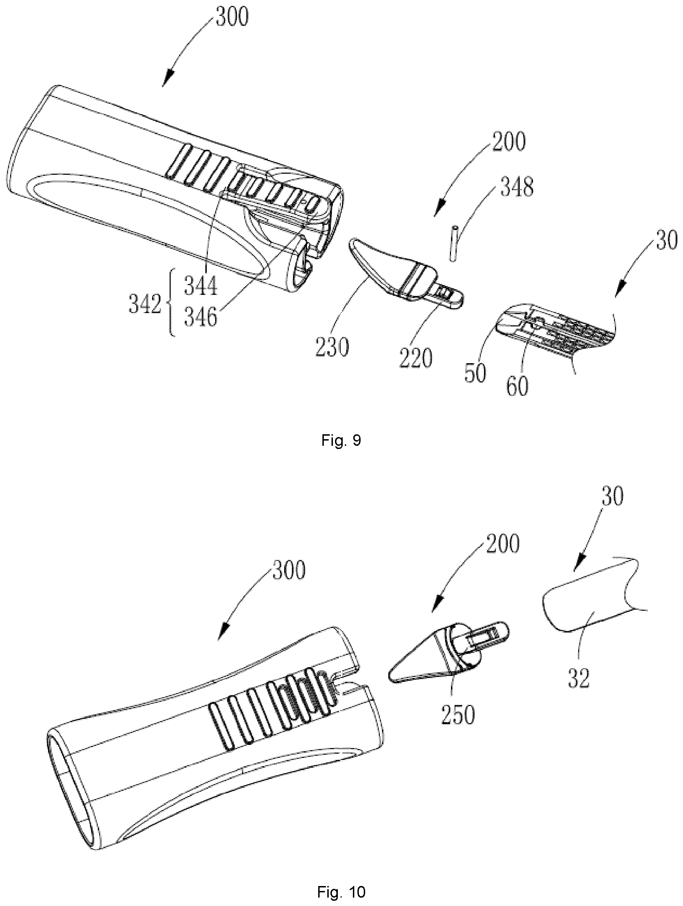

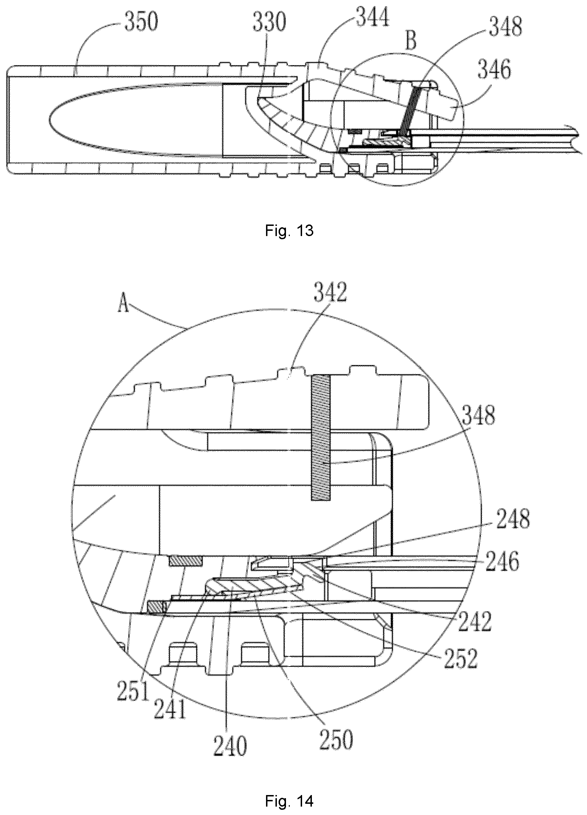

Fig. 2 to Fig. 15 show the separation member in a first implementation of the present invention. The separation member is used in cooperation with the surgical instrument 100.

Referring to Fig. 1 and Fig. 2, this implementation further provides the separation member 200 cooperatively used with the surgical instrument 100. The separation member 200 is used to strip the tissue. Specifically, the separation member 200 is used to strip the target tissue from other tissue, so that the surgical instrument 100 may conveniently cut and suture the target tissue. For example, a doctor first uses the separation member 200 to strip the blood vessel from other tissue, and then uses the surgical instrument 100 to cut and suture the stripped blood vessel.

The separation member 200 is detachably connected to the surgical instrument 100. That is to say, the separation member 200 switches between a first state and a second state. In the first state, the separation member 200 is connected to the surgical instrument 100; and in the second state, the separation member 200 is detached from the surgical instrument 100.

Therefore, when the separation member 200 is required to be used, the doctor connects the separation member 200 and the surgical instrument 100. The doctor first uses the separation member 200 to strip the target tissue from other tissue, and then moves the surgical instrument 100 forward, to cause the target tissue to be between the staple cartridge base 28 and the staple abutting seat 30, so as to operate the surgical instrument 100 to cut and suture the target tissue. When the separation member 200 is not required to be used, the doctor switches the separation member 200 from the first state to the second state, so that the separation member 200 is detached from the surgical instrument 100. In this way, the separation member 200 can be prevented from occupying surgical space to affect the convenience of the surgical instrument 100 for cutting and suturing other tissue.

Specifically, the separation member 200 is detachably connected to the end effector 18 of the surgical instrument 100. The end effector 18 is a working component in the surgical instrument 100 that is in direct contact with the tissue and cuts and sutures the tissue. That the separation member 200 is detachably connected to the end effector 18 conforms to the operation habits of the doctor and facilitates the operation of the doctor.

More specifically, the separation member 200 is detachably connected to the staple abutting seat 30 of the end effector 18. Since the staple abutting seat 30 of the conventional surgical instrument 100 is not equipped with the staple cartridge and other components, the separation member 200 is detachably connected to the staple abutting seat 30, so that the design is more rational by rationally using the staple abutting seat 30 without affecting the mounting and dismounting of other components (such as the staple cartridge). More specifically, the separation member 200 is detachably connected to a far end of the staple abutting seat 30, which is more conforms to the operation habits of the doctor and facilitates the operation of the doctor.

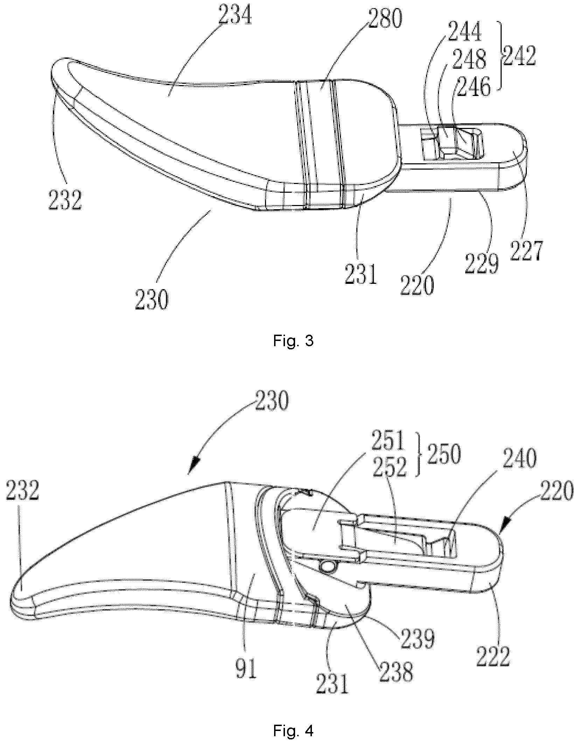

Referring to Fig. 3 to Fig. 5, the separation member 200 includes a mounting portion 220 and a working portion 230. The mounting portion 220 is detachably connected to the surgical instrument 100, and specifically, is detachably connected to the far end of the staple abutting seat 30 of the end effector 18. The working portion 230 is used to strip the tissue.

The mounting portion 220 is detachably connected to the surgical instrument 100 by using the mating mechanism. The mating mechanism includes an insertion mechanism and a clipping mechanism. The insertion mechanism includes an insertion portion 222 disposed to the separation member 200 and an insertion channel 50 disposed on the surgical instrument 100. The clipping mechanism includes a clipping portion 240 disposed on the separation member 200 and a clipping port 60 disposed on the surgical instrument 100. The insertion channel 50 communicates with the clipping port 60. Specifically, the clipping port 60 communicates with a near end of the insertion channel 50 and penetrates a sidewall of the insertion channel. When the separation member 200 is mounted, the insertion portion 222 and the clipping portion 240 are inserted into the surgical instrument 100 along the insertion channel 50. After a certain stroke is inserted, the clipping portion 240 is clipped in the clipping port 60, so that the separation member 200 is fixed on the surgical instrument 100. Therefore, the mounting portion 220 of the separation member 200 is detachably connected to the surgical instrument 100 through insertion and clipping, thereby achieving reliable mounting and convenient dismounting.

The mounting portion 220 includes the insertion portion 222 and the clipping portion 240 that are connected to each other. The clipping portion 240 is in elastically-mated connection with the surgical instrument 100. That is to say, the clipping portion 240 is connected to the surgical instrument 100 under the action of elastic force, and is detached from the surgical instrument 100 by overcoming the action of elastic force, so that a reliable connection and convenient dismounting can be realized. A source of the elastic force may be the elasticity of the clipping portion 240 itself. Alternatively, other elastomer may abut against the clipping portion 240, and provide the elastic force for the clipping portion 240.

Specifically, the clipping portion 240 is in elastically-mated connection with the clipping port 60. That is to say, the clipping portion 240 is clipped in the clipping port 60 under the action of elastic force, so that the separation member 200 is fixed to the surgical instrument 100. By overcoming the action of elastic force, the clipping portion 240 may be detached from the clipping port 60, so that the separation member 200 is detached from the surgical instrument 100.

The clipping portion 240 has a first end 241 and a second end 242. The first end 241 is connected to the insertion portion 222, and the second end 242 is in elastically-mated connection with the surgical instrument 100. Specifically, the second end 242 rotates around the first end 241, to cause the separation member 200 to be fixed to the surgical instrument 100 or to be detached from the surgical instrument, so as to achieve a detachable connection. The second end 242 rotates around the first end 241, that is, a rotation axis is located at a position of the first end 241. In this implementation, the rotation axis of the second end 242 is perpendicular to a central symmetry plane in a longitudinal direction of the separation member 200. In this implementation, the first end 241 is located on the far end relative to the second end 242. Those skilled in the art may think that, the first end 241 is located on the near end relative to the second end 242.

Specifically, the first end 241 includes a rotating shaft 243. The insertion portion 222 is provided with a shaft hole 224. The rotating shaft 243 is mounted in the shaft hole 224. The rotating shaft 243 rotates in the shaft hole 224 to cause the clipping portion 240 to rotate, so that the separation member 200 is finally detachably connected to the surgical instrument 100. The rotation axis is a central axis of the rotating shaft 243.

The shaft hole 224 is partially open, and the rotating shaft 243 is partially accommodated in the shaft hole 224. That is to say, a circumferential extension angle of the shaft hole 224 is less than 360 degrees. For example, the circumferential extension angle of the shaft hole 224 is 180 degrees. The shaft hole 224 is partially open, so that an operation of mounting the rotating shaft 243 into the shaft hole 224 is very convenient, and a size of the separation member 200 may also be reduced, to cause a structure of the separation member 200 to be more compact.

The first end 241 of the clipping portion 240 is mounted to a first side surface 229 of the insertion portion 222. It is to be noted that, the first side surface 229 refers to a surface of a back side of the insertion portion 222, and the surface is not only limited to a plane area indicated by 229. The second end 242 of the clipping portion 240 protrudes from a second side surface 227 of the insertion portion 222. It is to be noted that, the second side surface 227 refers to a surface of a front side of the insertion portion 222, and the surface is not only limited to a plane area indicated by 227. The insertion portion 222 is provided with an opening 228 penetrating the first side surface 229 and the second side surface 227. The second end 242 protrudes from the second side surface 227 by extending through the opening 228 from the first side surface 229.

Therefore, the opening 228 provides space for movement stroke of the second end 242 of the clipping portion 240. That is to say, a thickness of the insertion portion 222 provides space for the movement stroke of the second end 242 of the clipping portion 240. In this way, the structure is reliable; and the size of the separation member 200 may also be reduced, so that the design of the structure is very rational.

The second end 242 of the clipping portion 240 is in elastically-mated connection with the clipping port 60 of the surgical instrument 100. In this implementation, the clipping portion 240 itself has no elasticity. The separation member 200 further includes a support portion 250 abutting against the clipping portion 240. The support portion 250 elastically supports the clipping portion 240. The support portion 250 at least partially made of an elastic material. The support portion 250 provides elastic force for the clipping portion 240. That is to say, the clipping portion 240 only needs to realize a function of being engaged with or detached from the surgical instrument 100. The support portion 250 only needs to realize a function of providing the elastic force. The clipping portion 240 and the support portion 250 perform their own functions, so that process requirements for the clipping portion 240 and the support portion 250 can be reduced, which is more conform to mass production demand.

In order to apply acting force toward the second side surface 227 to the clipping portion 240, to cause the clipping portion 240 to be held in the clipping port 60 without external force, so as to be maintained in the first state of being connected to the surgical instrument 100, the support portion 250 is disposed on the first side surface 229 of the clipping portion 240 and is away from the second side surface 227 relative to the clipping portion 240.

Specifically, the support portion 250 has a first portion 251 and a second portion 252 that are connected to each other. The first portion 251 is connected to the first side surface 229. The second portion 252 abuts against the clipping portion 240, and provides the elastic force for the clipping portion 240, so as to cause the clipping portion 240 to have a trend to be clipped into the clipping port 60.

In order to cause the support portion 250 to be reliably fixed on the separation member 200, a connection portion 260 is disposed to the mounting portion 220. The support portion 250 and the mounting portion 220 are fixed through welding at the connection portion 260. Those skilled in the art may think that, other proper fixing method may also be used according to materials of the support portion 250 and the mounting portion 220.

As described above, the shaft hole 224 used for mounting the rotating shaft 243 is partially open. In order to prevent the rotating shaft 243 from falling off the open shaft hole 224, the support portion 250 covers the open portion of the shaft hole 224. Specifically, the connection portion 260 is disposed adjacent to the shaft hole 224. The connection portion 260 is away from the second end 242 of the clipping portion 240 relative to the shaft hole 224. The far end of the first portion 251 of the support portion 250 is fixed on the connection portion 260. Therefore, the support portion 250 extending from the far end to the near end may naturally cover the shaft hole 224, so that the design of the structure is very rational.

It may be seen that, a position of one portion of the rotating shaft 243 is defined by the shaft hole 224, and a position of the other portion is defined by the support portion 250. Compared with the support portion 250 superposed on the totally-closed shaft hole 224, the size of the separation member 200 can be obviously reduced, so that the structure of the separation member 200 can be more compact.

It is to be noted that, the present invention focuses on reducing the size of the separation member 200 in a plurality of aspects. Although the reduction in size of these designs may not be noticeable to naked eyes (for example, less than 1 mm), the size of the separation member 200 is critical. The reason is that: the end effector 18 of the surgical instrument 100 and the separation member 200 are required to enter the body of the patient by extending through the cannula of the puncture outfit for operation; in order to achieve the purpose of minimally invasive surgery, the cannula of the puncture outfit is available in standard sizes (for example, 5 mm/10 mm/12 mm/15 mm); and widths of the end effector 18 and the separation member 200 must be less than a diameter of the cannula of the puncture outfit, so that when designing the end effector 18 and the separation member 200, a designer needs to repeatedly consider and optimize the structure to achieve the purpose even if the size is reduced by less than 1 mm.

As described above, the first end 241 of the clipping portion 240 is mounted to the first side surface 229 of the insertion portion 222. The first portion 251 of the support portion 250 is connected to the first side surface 229. In order to better arrange the clipping portion 240 and the support portion 250, the first side surface 229 of the insertion portion 222 is recessed inward to form accommodating space 226. That is to say, the accommodating space 226 is disposed at the insertion portion 222 of the mounting portion 220, and partial clipping portion 240 is accommodated in the accommodating space 226. The accommodating space 226 includes the opening 228 penetrating the insertion portion 222. The second end 242 of the clipping portion 240 protrudes from the opening 228 to be in clipped connection with the clipping port 60 in the first state. When the separation member 200 is in the first state, partial clipping portion 240 is accommodated in the accommodating space 226. Preferably, when the separation member 200 is in the first state, partial support portion 250 is also accommodated in the accommodating space 226.

Since the accommodating space 226 may store at least partial clipping portion 240, or even store partial support portion 250, the clipping portion 240 and the support portion 250 are prevented from completely protruding from the first side surface 229 of the insertion portion 222. By rationally using the space of the insertion portion 222 itself, not only the size of the insertion portion 222 can be reduced, but also the structure of the insertion portion 222 can be more regular. The accommodating space 226 accommodates partial clipping portion 240, and may also achieve limitation to the clipping portion 240. The second end 242 of the clipping portion 240 is located in the opening 228, so that the second end 242 may change the position under the action of the elastic force to achieve its function. Preferably, partial support portion 250 is accommodated in the accommodating space 226; and the support portion may be prevented from interfering with the insertion channel when the insertion portion is inserted into the insertion channel.

In this implementation, the insertion portion 222 and the clipping portion 240 have no elasticity. The separation member 200 further includes the elastomer abutting against the clipping portion 240. The elastomer abuts against the support portion 240 to elastically support the clipping portion 240. The elastomer is the support portion 250. The support portion 250 at least partially made of an elastic material. The support portion 250 provides elastic force for the clipping portion 240. Those skilled in the art may think that, the clipping portion 240 may also have the elasticity, and any solution that is the same as or similar to this implementation is covered within the protection scope of the present invention.

In this implementation, there is no other elastomer between the support portion 250 and the clipping portion 240, and the elastic force is provided for the clipping portion 240 only by the support portion 250. That is to say, the elastomer abutting against the clipping portion 240 and elastically supporting the clipping portion 240 only includes the support portion 250. Those skilled in the art may think that, on the basis of disposing the support portion 250, another elastomer is disposed between the support portion 250 and the clipping portion 240, for example, second elastomer. The second elastomer and the support portion 250 provide the elastic force for the clipping portion 240 together. Alternatively, the support portion 250 is not disposed, and the elasticity may also be provided for the clipping portion 240 only by other elastomer. Any solution that is the same as or similar to this implementation is covered within the protection scope of the present invention.

The second end 242 of the clipping portion 240 includes a clipping surface 244 and a slope 246 disposed with the clipping surface 244 at an angle. As shown in Fig. 3, the clipping surface 244 extends vertically. As shown in Fig. 2, Fig. 3 and Fig. 6, the clipping surface 244 abuts against the sidewall of the clipping port 60 to prevent the separation member 200 from falling off the surgical instrument 100. As shown in Fig. 3, the slope 246 obliquely extends upward in a direction from the near end to the far end. The upward here refers to a direction toward the opening of the clipping port 60. Under the guide of the slope 246, the position of the second end 242 is changed due to the rotation of the clipping portion 240 in a process of being inserted into the insertion channel 50, so that the insertion portion 222 of the separation member 200 and the clipping portion 240 may be inserted into the insertion channel 50 more smoothly in the direction from the far end to the near end.

The second end 242 of the clipping portion 240 further includes a top surface 248 disposed between the clipping surface 244 and the slope 246. The top surface 248 has a certain width, and may cause the structure of the second end 242 to be more reliable. The top surface 248 may be a straight surface, or may be the inclined slope 246. If the top surface 248 is a slope, an inclined angle of the top surface 248 is preferably less than an inclined angle of the slope 246.

It is to be noted that, in this implementation, the clipping portion 240 is integrally formed. Those skilled in the art may think that, the portions of the clipping portion 240 are independently formed and are connected in a specific connection manner. Any solution that is the same as or similar to this implementation is covered within the protection scope of the present invention.

As described above, the mounting portion 220 of the separation member 200 includes the insertion portion 222 for mounting the clipping portion 240. Referring to Fig. 4 to Fig. 6, the insertion portion 222 extends lengthwise. Correspondingly, the insertion channel 50 extends from the far end surface of the surgical instrument 100 in a direction toward the near end. Specifically, the insertion channel 50 extends from the far end surface of the staple abutting seat 30 in the direction toward the near end.

It is well known to those skilled in the art that, the staple abutting seat 30 is provided with a knife-moving slot 33 extending lengthwise. The knife-moving slot 33 stores partial cutting component of the surgical instrument 100 and guides the movement of the cutting component. The knife-moving slot 33 is disposed in a center position of the staple abutting seat 30 and extends lengthwise. The insertion channel 50, the clipping port 60 and the knife-moving slot 33 communicate with each other and are coaxially disposed, so that a regular structure is achieved.

The clipping port 60 communicates with the near end of the insertion channel 50 and penetrates the sidewall of the insertion channel 50. Specifically, the clipping port 60 penetrates the sidewall of a staple against surface 31 of the staple abutting seat 30.

Therefore, when the separation member 200 is required to be mounted to the surgical instrument 100, the insertion portion 222 of the separation member 200 is aligned to the insertion channel 50, and the separation member 200 is pushed in the direction from the far end to the near end. During pushing, the insertion channel 50 abuts against the slope 246 of the second end 242 of the clipping portion 240, so that the clipping portion 240 rotates by overcoming the elastic force provided by the support portion 250, until the second end 242 of the clipping portion 240 and the insertion portion 222 are successfully inserted into the insertion channel 50. The separation member 200 is continuously pushed, until the second end 242 of the clipping portion 240 is flush with the clipping port 60. The second end 242 of the clipping portion 240 rotates in a reverse direction under the action of the elastic force provided by the support portion 250, and the second end 242 protrudes from the second side surface 227 of the insertion portion 222 and is clipped in the clipping port 60. In this way, the separation member 200 is mounted in place.

The sidewall of the insertion channel 50, specifically, the sidewall of the insertion channel 50 located on the staple against surface 31 of the staple abutting seat 30 includes a first excavated portion 51 and a second excavated portion 52 that communicate with the clipping port 60. The second excavated portion 52 is located on the far end relative to the first excavated portion 51. A width of the second excavated portion 52 is greater than a width of the first excavated portion 51. A width of the clipping port 60 is greater than the width of the first excavated portion 51.

During the inserting of the separation member 200, the wider second excavated portion 52 may cause the insertion operation of the separation member 200 to be easier. During the moving of the separation member 200 in the second excavated portion 52, the clipping portion 240 is not required to overcome the elasticity of the support portion 250 to rotate, and the support portion 250 is not required to be deformed, so that the service life of the separation member 200 may be prolonged, and the increasing of resistance due to the contact between the clipping portion 240 and the second excavated portion may also be prevented. When the slope 246 of the second end 242 of the separation member 200 is in contact with the first excavated portion 51, the second end 242 of the clipping portion 240 may overcome the elasticity of the support portion 250 to rotate and is moved inside the first excavated portion 51. When the separation member 200 is flush with the clipping port 60 by moving to the second end 242 of the clipping portion 240, since the width of the clipping port 60 is greater than the width of the first excavated portion 51, the clipping surface 244 of the clipping portion 240 may abut against the sidewall of the first excavated portion 51 adjacent to the clipping port 60. In addition, the second end 242 of the clipping portion 240 rotates in the reverse direction under the action of the elastic force of the support portion 250 to protrude from the second side surface 227, so that the second end 242 can be successfully clipped in the clipping port 60, and the separation member 200 is mounted in place.

It may be seen that, through the rational design of the first excavated portion 51 and the second excavated portion 52, not only the service life of the separation member 200 can be prolonged, but also the mounting operation of the separation member 200 can be easier and more convenient.

A length of the second excavated portion 52 is greater than a length of the first excavated portion 51. That is to say, during the movement of the clipping portion 240 in the second excavated portion 52, the clipping portion 240 is not required to overcome the elasticity of the support portion 250 to rotate, and the support portion 250 is not deformed, so that the service life of the separation member 200 can be prolonged.

The width of the first excavated portion 51 equals to a width of the knife-moving slot 33. The knife-moving slot 33, the clipping port 60, the first excavated portion 51 and the second excavated portion 52 are coaxially disposed, so that a proper position of the separation member 200 after mounting may be guaranteed, the separation member does not deviate from the central axis, and the doctor may conveniently operate the separation member.

The second excavated portion 52, the first excavated portion 51, the clipping port 60, and even the knife-moving slot 33 form a channel groove in the sidewall of the insertion channel 50 extending from the far end to the near end.

A first wedge portion 39 is disposed on the far end of the staple abutting seat 30. The first wedge portion 39 has a first slope 38. A second wedge portion 239 is disposed on the near end of the working portion 230. The second wedge portion 239 has a second slope 238. After the separation member 200 is mounted to the staple abutting seat 30, the first slope 38 abuts against the second slope 238.

Since the first slope 38 abuts against the second slope 238, the first wedge portion 39 is superposed with the second wedge portion 239, so that the separation member 200 may be mounted on the staple abutting seat 30 more reliably. In addition, the first wedge portion 39 may cause the far end of the staple abutting seat 30 to be thinner relative to other portions. The second wedge portion 239 may cause the near end of the working portion to be thinner relative to other portions. That is to say, even if the first wedge portion 39 is superposed with the second wedge portion 239, a size of the junction is not obviously increased. In an aspect, the superposition causes the mounting to be reliable; and in another aspect, the size is not obviously increased by the superposition, so that the design is very rational.

The second excavated portion 52 is disposed to the first slope 38. The mounting portion 220 extends from the second slope 238 in the direction toward the near end, so that the structure is compact, and the design is rational.

The combined thickness of the first wedge portion 39 and the second wedge portion 239 is equivalent to the thickness of the staple abutting seat 30 adjacent to the near end of the first slope 38, and/or is equivalent to the thickness of the separation member adjacent to the far end of the second slope 238.

Since the thicknesses are equivalent, that is, the thicknesses are approximately equal, a step is prevented from appearing at the junction of the separation member 200 and the staple abutting seat 30, so that the tissue can be prevented from being scratched.

As described above, the separation member 200 includes the mounting portion 220 and the working portion 230. The working portion 230 is used to strip the tissue.

The working portion 230 includes the near end 231 and a far end 232. In a direction from the near end 231 to the far end 232, a width of the working portion 230 gradually decreases. When the working portion 230 strips the tissue, the narrow far end 232 preferentially contacts the tissue, so that the narrow far end 232 may be conveniently inserted into the tissue and strip the target tissue from other tissue, thereby achieving more convenient operation.

At least a portion of the working portion 230 extends in an arc line from the near end 231 to the far end 232. The working portion 230 has a working surface 234 extending in the arc line and recessed inward. By means of the working surface 234 extending in the arc line, the working portion 230 may successfully pass through a gap between the target tissue and other tissue when stripping the tissue, to better guide the end effector 18 to pass through the gap between the target tissue and other tissue, so that the separation member 200 and the surgical instrument 100 can be more convenient in operation.

The working portion 230 extends in the arc line, and the working surface 234 extends in the arc line, so that the back side of a working member also extends in the arc line. The working portion 230 is regular in structure and good-looking in appearance.

Outer contour lines of the working portion 230 and the mounting portion 220 of the separation member 200 have not obvious edges and corners. All of the outer contour lines are rounded, so that the edges and corners may be prevented from cutting the tissue.

As described above, the separation member 200 is mounted on the far end of the surgical instrument 100. The surgical instrument 100 has a mounting end used for mounting the separation member 200. The separation member 200 further includes an elastic member 280. The elastic member 280 abuts against an end surface of the mounting end. Specifically, the elastic member 280 abuts against the end surface of the far end of the staple abutting seat 30.

Since the end surface of the far end of the mounting end is hard and the elastic member 280 is flexible and deformable, when the elastic member 280 abuts against the end surface of the far end of the mounting end, the end surface of the far end of the mounting end may be prevented from scratching the tissue, and the risk that the tissue is sandwiched in a gap between the end surface of the far end of the mounting end and the separation member 200 may also be reduced, so that unnecessary pulling to the tissue can be avoided, thereby preventing the tissue from being damaged.

An outer surface of the elastic member 280 protrudes from an outer surface of the mounting end or is flush with the outer surface of the mounting end. Specifically, the staple abutting seat has the staple against surface 31 and a back surface 32. In this implementation, preferably, the outer surface of the elastic member 280 is flush with the back surface 32 of the staple abutting seat 30 or protrudes from the back surface 32. When the target tissue is stripped from other tissue, since the mounting end on the side of the back surface 32 of the staple abutting seat 30 is easy to scratch other tissue, the outer surface of the elastic member 280 is flush with the back surface 32 of the staple cartridge or protrudes from the back surface 32, so that the end surface of the far end of the mounting end may be prevented from scratching the tissue to the greatest extend, and the risk that the tissue is sandwiched in the gap between the end surface of the far end of the mounting end and the separation member 200 may be reduced to the greatest extend.

The separation member 200 is provided with a slot 290. The mounting end has a first outer surface 91, and the separation member 200 has a second outer surface 202. When the separation member 200 is in the first state, the slot 290 is located at an adjacent portion of the first outer surface 91 and the second outer surface 202. The elastic member 280 is disposed in the slot 290. By using the slot 290, the elastic member 280 may be better mounted without affecting the functions of the elastic member 280.

The outer surface of the elastic member 280 protrudes from the second outer surface 202 or is flush with the second outer surface 202. By using the slot 290, the elastic member 280 may be better mounted, but, there is a risk of scratching the tissue at the near end and/or the far end of the slot 290. By causing the outer surface of the elastic member 280 to protrude the second outer surface 202 or to be flush with the second outer surface 202, the elastic member 280 can be conveniently mounted, and the tissue can also be prevented from being scratched.

A shape of the elastic member 280 matches a shape of the slot 290, so that the tissue can be prevented from being scratched to the greatest extent. The elastic member 280 is in interference fit with the slot 290, so that the elastic member 280 can be mounted reliably, and the elastic member 280 can conveniently protrude from the surface adjacent to the elastic member, thereby preventing the tissue from being scratched to the greatest extent.

The slot 290 extends circumferentially. Therefore, the mounting of the elastic member 280 may be more reliable.

A bottom surface of the slot 290 is rough. Specifically, the bottom surface of the slot includes depressions and/or projections, which may increase the friction force between the slot 290 and the elastic member 280. In this way, the elastic member 280 can be reliably mounted in the slot 290.

A shape of the bottom surface of the slot 290 is symmetrically designed relative to the central symmetry plane in the longitudinal direction of the separation member 200. Since the structure is regular, the elastic member 280 is evenly stressed, so that an effect of preventing tissue scratching can be better achieved.

In this implementation, the elastic member 280 is a component formed independently. The elastic member 280 is manually disposed in the slot 290. Those skilled in the art may think that, the elastic member 280 may also be formed in the slot 290 in a spraying manner. Any solution that is the same as or similar to this implementation is covered within the protection scope of the present invention.

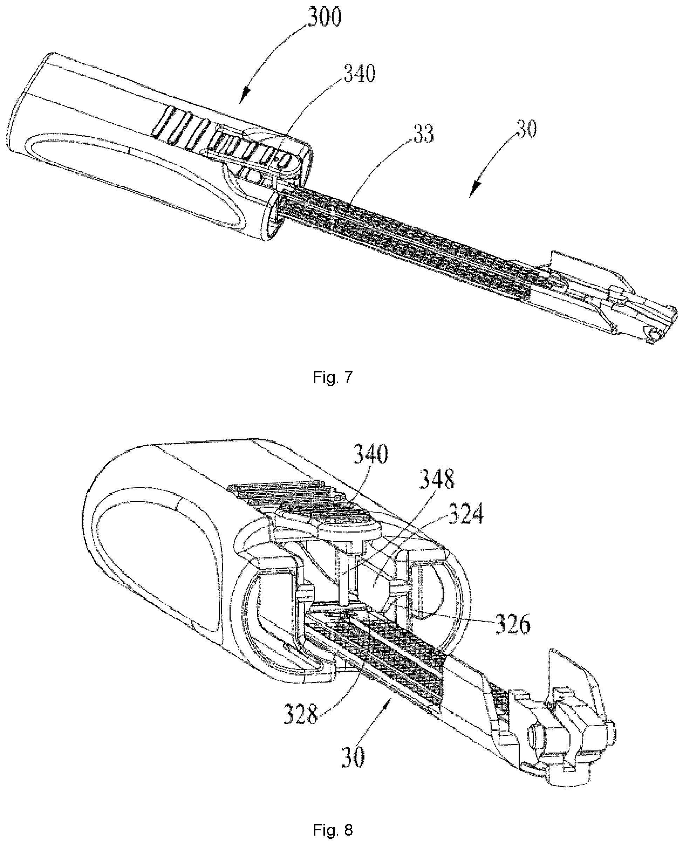

Referring to Fig. 7 to Fig. 13, this implementation further provides a packaging box 300 of the separation member 200. The separation member 200 may be accommodated in the packaging box 300. The packaging box 300 provides an external packing for the separation member 200, so that the separation member 200 is conveniently transported and stored. The separation member 200 and the packaging box 300 form an assembly. The assembly may be sold and purchased separately, or may be sold and purchased together with the surgical instrument 100 to form a surgical kit. Regardless of sale and purchase methods, the needs of the doctor to strip the target tissue can be met. Definitely, the separation member 200 in this implementation may also be sold and purchased separately.

The packaging box 300 includes an accommodation portion 320 used for accommodating the separation member 200. The separation member 200 may be accommodated in the packaging box 300 by inserting the accommodation portion 320. The packaging box 300 includes a housing 350. A surface of a side of the housing 350 is recessed inward to form the open accommodation portion 320. The accommodation portion 320 has an open end, so that the separation member 200 can be conveniently put in or taken out from the packaging box 300 from the open end.

The accommodation portion 320 has a closed end 330 spaced apart from the open end. A shape of the closed end 330 may match a shape of the far end 232 of the separation member 200. The shape of the closed end 330 may also not match the shape of the far end 232 of the separation member 200, as long as the closed end 330 may limit the inserting of the separation member 200. Therefore, during the inserting of the separation member 200 into the accommodation portion 320, when the far end 232 of the separation member 200 abuts against the closed end 330 of the accommodation portion 320, an operator can perceive that the separation member 200 has been accommodated in place.

The accommodation portion 320 is provided with a convex rib 324. The convex rib 324 is provided with a guide surface 326. The separation member 200 slides into the accommodation portion 320 along the guide surface 326. The guide surface 326 may cause the separation member 200 to be inserted into the accommodation portion 320 more easily and accurately.

The convex rib 324 is further provided with an abutment surface 328. The abutment surface 328 and the guide surface 326 intersect with each other and are disposed at an angle, specifically, at an obtuse angle. The abutment surface 328 is close to the closed end 330 of the accommodation portion 320 relative to the guide surface 326. The abutment surface 328 limits the separation member 200. Specifically, when the separation member 200 is mounted in the accommodation portion 320, the abutment surface 328 abuts against partial working surface 234 of the separation member 200, so that the separation member 200 can be accommodated in the accommodation portion 320 more reliably.

It may be seen that, the convex rib 324 may not only define the size of the accommodation portion 320, but also guide the operation of the separation member 200 to slide into the accommodation portion 320. After the separation member 200 slides into the accommodation portion 320, the convex rib 324 may also limit the separation member 200, so that the design of the structure is very rational.

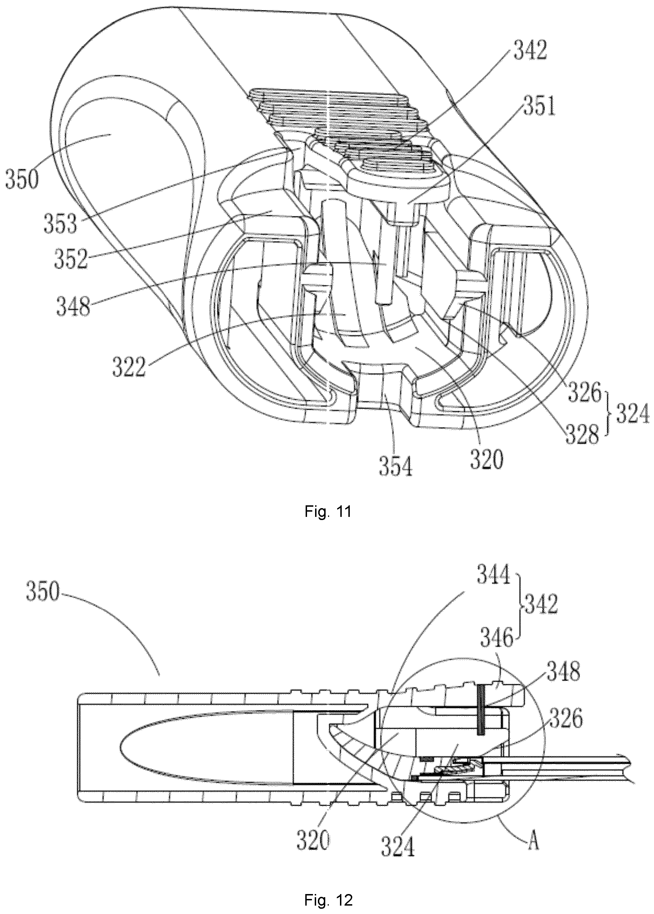

Referring to Fig. 11, a protrusion 322 is disposed on a surface of the accommodation portion 320. The protrusion 322 may support the working portion 230 of the separation member 200, and reduce a contact area between the working portion 230 and the accommodation portion 320, so that the separation member 200 can be inserted or taken out more easily. In this implementation, the protrusion 322 extends lengthwise and is approximately in a strip shape. The protrusion 322 extends in a longitudinal direction. Those skilled in the art may think that, the protrusion 322 may be in other shapes.

As described above, the separation member 200 includes the elastic member 280 abutting against the end surface of the mounting end of the surgical instrument 100. Preferably, the outer surface of the elastic member 280 protrudes from the outer surface of the working portion 230 of the separation member 200. Therefore, the elastic member 280 elastically abuts against an inner surface of the accommodation portion 320.

During the inserting of the separation member 200 into the accommodation portion 320 of the packaging box 300, and when the elastic member 280 elastically abuts against the inner surface of the accommodation portion 320, the operator can perceive the effect of damping. The effect of damping provides operation feedback for the operator, so that the operator may determine whether the separation member 200 is accommodated in place by means of such feedback. That is to say, in addition to the foregoing closed end 330 allowing the operator to perceive that the separation member 200 has been inserted in place, that the elastic member 280 elastically abuts against the inner surface of the accommodation portion 320 further provides feedback for the operator, thereby further improving operation experience. In addition, the friction force between the elastic member 280 and the inner surface of the accommodation portion 320 may prevent the separation member 200 from falling off the accommodation portion 320.

As described above, the separation member 200 is detachably connected to the surgical instrument 100 by using a mating mechanism. The separation member 200 switches between a first state and a second state. In the first state, the separation member 200 is connected to the surgical instrument 100; and in the second state, the separation member 200 is detached from the surgical instrument 100.

In this implementation, preferably, the packaging box 300 may not only accommodate the separation member 200, but also switch the separation member 200 between the first state and the second state. That is to say, the packaging box 300 can not only provide packaging for the separation member 200, but also serve as a disassembling tool of the separation member 100 relative to the surgical instrument 200, of which design is very artful.

Specifically, the packaging box 300 includes an operating member 340. The operating member 340 acts on the mating mechanism to switch the separation member 200 from the first state to the second state. More specifically, the operating portion 342 acts on the clipping mechanism to switch the separation member 200 from the first state to the second state.

The operating member 340 includes the operating portion 342 and a driving portion 348 that are connected to each other. The operating portion 342 is pressed by the operator. The driving portion 348 acts on the clipping mechanism to switch the separation member 200 from the first state to the second state.

The operating portion 342 performs first movement to drive the driving portion 348 to perform second movement, so as to switch the separation member 200 from the first state to the second state. The first movement includes rotation; and the second movement includes rotation.

The packaging box 300 includes the housing 350. The housing 350 is partially recessed inward to form the accommodation portion 320. The housing 350 includes a first sidewall 351 and a second sidewall 352 surrounding the accommodation portion 320. There is a gap 353 between the first sidewall 351 and the second sidewall 352. The first sidewall 351 forms the operating portion 342. The gap 353 extends lengthwise in a direction from one end of the housing 350 to the other end. An extending length of the gap 353 is less than an extending length of the housing 350. Due to the presence of the gap 353, the first sidewall 351 may move relative to the second sidewall 352, so that the driving portion 348 is driven to move and finally acts on the separation member 200 to switch the separation member 200 from the first state to the second state. In addition, the first sidewall 351 is a portion of the housing 350. A portion of the housing 350 forms the operating portion 342, and other components are not required to be extra disposed to form the operating portion 342. Therefore, a simple structure and rational design can be achieved.

The operating portion 342 rotates to drive the driving portion 348 to rotate. Specifically, the first sidewall 351 includes a connection end 344 and a free end 346. The driving portion 348 is connected to the free end 346. The free end 346 rotates around the junction of the connection end 344 and the second sidewall to drive the driving portion 348 to rotate. Extra components are not required to be disposed, so that the simple structure and rational design can be achieved.

A rotation axis of the free end 346 is perpendicular to a central symmetry plane in a longitudinal direction of the packaging box 300. The rotation axis of the free end 346 is also parallel to a rotation axis of the clipping portion 240. Therefore, the assembly formed by the separation member 200 and the packaging box 300 may be regular in structure.

The connection end 344 of the operating portion 342 is made of an elastic material. Optionally, the entire operating portion 342 is made of the elastic material. Therefore, when the free end 346 is pressed, the connection end 344 is deformed, to cause the free end 346 to rotate around the junction of the connection end 344 and the second sidewall. Therefore, the doctor overcomes the elastic force of the operating portion 342 itself to drive the driving portion 348 to move, so that the separation member 200 may be switched from the first state to the second state. By using the elasticity of the operating portion 342 itself, the structure of the packaging box 300 can be simpler. By means of the elasticity of the operating portion 342 itself, the free end 346 is maintained in a raised state without external force, as shown in Fig. 8, Fig. 11 and Fig. 12.

Both the operating portion 342 and the driving portion 348 extend lengthwise. An extending direction of the operating portion 342 is perpendicular to an extending direction of the driving portion 348. The operating portion 342 and the driving portion 348 that extend lengthwise and have the mutually perpendicular extending directions provide an enough stroke, so that the driving portion 348 may effectively the clipping portion 240. Therefore, the separation member 200 can be successfully detached from the surgical instrument 100.

The operating portion 342 is connected to the driving portion 348, and cooperatively abuts against the clipping portion 240. The operating portion 342 and the driving portion 348 are two independent components. Specifically, the driving portion 348 is a pin, and the pin is mated with a hole in the operating portion 342 to achieve the connection between the driving portion 348 and the operating portion 342. Those skilled in the art may think that, the operating portion 342 and the driving portion 348 may also be integrally formed.

As shown in Fig. 11 and Fig. 12, the housing 350 is provided with an opening hole 354 for the driving portion 348 to pass through. The opening hole 354 is disposed opposite to the driving portion 348. Therefore, when the driving portion 348 is mounted, the driving portion 348 is connected to the operating portion 342 after extending through the opening hole 354. Through the opening hole 354, a mounting tool can conveniently extend to apply force to the driving portion 348, so that the structure is rational in design. The driving portion 348 is connected to the free end 346 of the operating portion 342. Correspondingly, the opening hole 354 is opposite to the free end 346 of the operating portion 342. Therefore, rational layout can be achieved.

A width of the driving portion 348 is less than a width of the channel groove (including the second excavated portion 52, the first excavated portion 51 and the clipping port 60 that successively communicate with each other) in the staple abutting seat 30. During the detaching of the separation member 200 from the surgical instrument 100, the driving portion 348 may be moved in the channel groove and maintained in a state of pressing the second end 242 of the clipping portion 240.

It may be learned that, the operating member 340 acts on the clipping mechanism to switch the separation member 200 from the first state to the second state.

The far end of the accommodation portion 320 is closed to form the closed end 330. When the separation member 200 is mounted to the surgical instrument 100, the packaging box 300 accommodating the separation member 200 drives the separation member 200 to move toward the surgical instrument 100. The closed end 330 pushes the separation member 200 to insert into the surgical instrument 100. Therefore, the closed end 330 of the accommodation portion 320 of the packaging box 300 pushes the separation member 200 to switch from the second state to the first state.

The packaging box 300 is made of the transparent material, so that the packaging box is good-looking in appearance. When the separation member 200 is dismounted by using the packaging box 300, the transparent packaging box 300 facilitates the operator to observe whether the dismounting operation is in place.

An application scenario of the separation member 200 in this implementation is described below.

After the separation member 200 leaves factory and before the separation member is used, the separation member 200 is located in the accommodation portion 320 of the packaging box 300. The elastic member 280 on the separation member 200 elastically abuts against the inner side surface of the accommodation portion 320. The separation member 200 is held in the packaging box 300 under the action of the elastic force of the elastic member 280.

When the doctor needs to use the separation member 200 to strip the tissue during the using of the surgical instrument 100, the doctor holds the housing 350 of the packaging box 300, to align the mounting portion 220 of the separation member 200 with the insertion channel 50 disposed on the staple abutting seat 30 of the end effector 18 of the surgical instrument 100, and pushes the packaging box 300 in a direction toward the surgical instrument 100. During pushing, the insertion portion 222 of the separation member 200 and the clipping portion 240 are moved along the insertion channel 50, until the second end 242 of the clipping portion 240 is flush with the clipping port 60 and clipped in the clipping port 60. In this case, the separation member 200 has been mounted on the surgical instrument 100.

Then, the doctor overcomes the friction force between the elastic member and the inner side surface of the accommodation portion 320 due to the elastic force of the elastic member 280, to pull the packaging box 300 in a direction away from the surgical instrument 100, so as to detach the packaging box 300 from the separation member 200, so that the doctor may use the separation member 200 to strip the target tissue. Specifically, the narrow far end 232 of the working portion 230 of the separation member 200 is aligned with an interface of the target tissue and other tissue, and the surgical instrument 100 and the separation member 200 are pushed forward, until the separation member 200 strips the target tissue from other tissue.

After the separation member 200 strips the target tissue from other tissue, the doctor moves forward the surgical instrument 100 in a direction toward the far end 232, to cause the target tissue to be between the staple abutting seat 30 and the staple cartridge base. Then, the doctor may use the surgical instrument 100 to cut and suture the target tissue.

When the doctor does not need to strip the tissue during the using of the surgical instrument 100, the separation member 200 may be taken down from the surgical instrument 100, to prevent the separation member 200 from occupying space to affect other operations when the surgical instrument 100 does not need the separation member 200. Specifically, the doctor aligns the packaging box 300 with the separation member 200 to move to accommodate the separation member 200 in the accommodation portion 320 of the packaging box 300. Then, the doctor presses the free end 346 of the operating portion 342, and then the free end 346 rotates around the connection end 344 to drive the driving portion 348 to rotate, until the driving portion 348 abuts against the slop 246 or the top surface 248 of the second end 242 of the clipping portion 240. The free end 346 of the operating portion 342 is continuously pressed, and the driving portion 348 drives the second end 242 of the clipping portion 240 to rotate against the acting force of the support portion 250, until the second end 242 of the clipping portion 240 is detached from the clipping port 60. Then, the doctor pulls the packaging box 300 in the direction away from the surgical instrument 100. The packaging box 300 drives the insertion portion 222 of the separation member 200 to be detached from the insertion channel 50, so that the separation member 200 is detached from the surgical instrument 100. The separation member 200 detached from the surgical instrument 100 is located in the accommodation portion 320 of the packaging box 300, which returns back to a state before the separation member is used and after the separation member leaves factory. It is to be noted that, after the driving portion 348 causes the second end 242 of the clipping portion 240 to be detached from the clipping port, the driving portion 348 maintains the state of pressing the second end 242 of the clipping portion 240. That is to say, during the detaching of the separation member 200 from the surgical instrument 100, the driving portion 348 presses the second end 242 of the clipping portion 240 all the time and applies force to the second end 242. The driving portion 348 moves in the channel groove. Finally, the packaging box 300 drives the separation member 200 to detach from the surgical instrument 100.

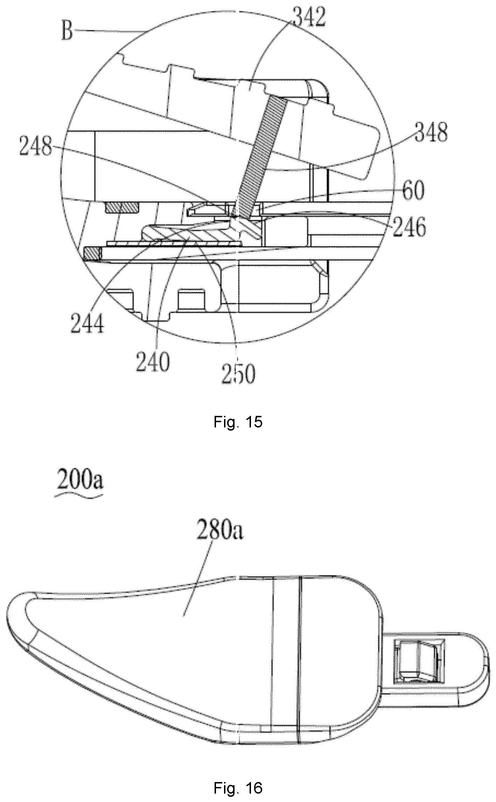

Fig. 16 to Fig. 17 show the separation member 200a cooperatively used with the surgical instrument provided in a second implementation of the present invention.

A difference between the separation member 200a of this implementation and the separation member in the first implementation is mainly described below.

In the first implementation, the elastic member circumferentially extends into an annular shape. The annular elastic member is mounted in a slot between the separation member and the far end surface of the staple abutting seat.

In this implementation, the elastic member 280a is in a bag shape. At least a portion of the working portion 230a is inserted in the elastic member 280a. The at least a portion of the working portion 230a has a same shape as the elastic member 280a. Specifically, the working portion 230a is inserted in the elastic member 280a from the far end to a portion adjacent to the mounting end of the surgical instrument. The end surface of the opening on the near end of the elastic member 280a abuts against the far end surface of the mounting end, to prevent the far end surface of the mounting end from scratching the tissue.

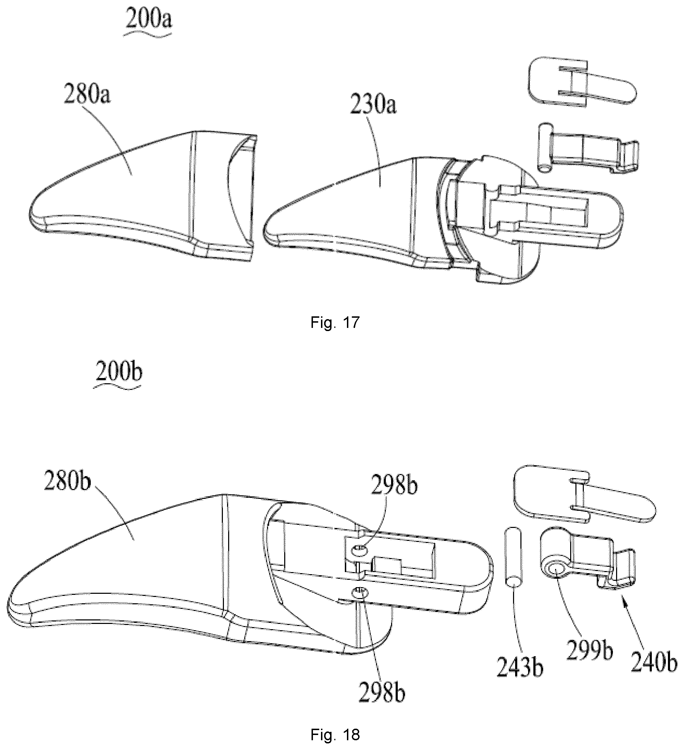

Fig. 18 shows the separation member cooperatively used with the surgical instrument provided in a third implementation of the present invention.

A difference between the separation member 200b of this implementation and the separation member in the first implementation is mainly described below.

In the first implementation, a first end of the clipping portion includes a rotating shaft. The rotating shaft and other portions of the clipping portion are integrally formed. The insertion portion is provided with an open shaft hole. The rotating shaft is partially accommodated in the shaft hole. The support portion covers the open portion of the shaft hole, so that a second end of the clipping portion can rotate around the rotating shaft.

In this implementation, the separation member 200b includes an independent rotating shaft 234b. The first end of the clipping portion 240b is provided with a pin hole 299b extending through the rotating shaft 234b. The mounting portion of the separation member 200b is provided with circular holes 298b corresponding to two end portions of the pin hole 299b. The rotating shaft 234b successively extends through the first circular hole 298b, the pin hole 299b and the second circular hole 298b, so that the first end of the clipping portion 240b is rotatably mounted on the mounting portion. The rotating shaft 234b is mated with the first circular hole 298b and/or second circular hole 298b, to prevent the rotating shaft from falling off.

In addition, in this implementation, the shape of the elastic member 280b of the separation member 200b, the elastic member 280b and the mating mechanism of the working portion 230b are the same as that in the second implementation, which are not described again.

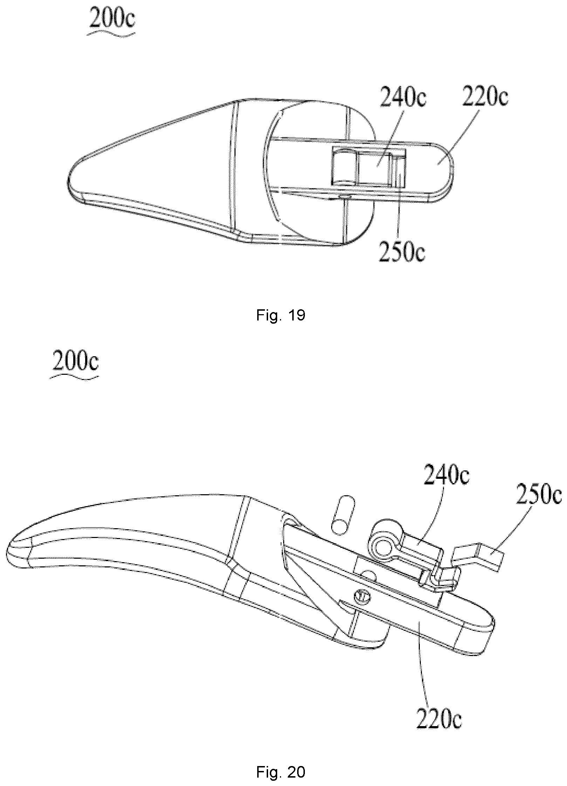

Fig. 19 to Fig. 20 show the separation member cooperatively used with the surgical instrument provided in a fourth implementation of the present invention.

A difference between the separation member 200c of this implementation and the separation member in the third implementation is mainly described below.

In the third implementation, the connection portion is disposed on a far side of the mounting portion of the separation member adjacent to the first end of the clipping portion. The first portion of the support portion is connected to the connection portion. The support portion extends toward the near end from the connection portion. The support portion applies the force to the clipping portion relative to the second portion of the near end, so that the clipping portion has a trend to be clipped in the clipping port of the surgical instrument.

In this implementation, the support portion 250c is disposed close to the near end of the clipping portion 240c. The near end of the support portion 250c is connected to the mounting portion 220c in a manner of welding. The support portion 250c has elasticity. The far end of the support portion 250c applies the force to the second end of the clipping portion 240c, so that the clipping portion 240c has a trend to be clipped in the clipping port 60c in the surgical instrument.

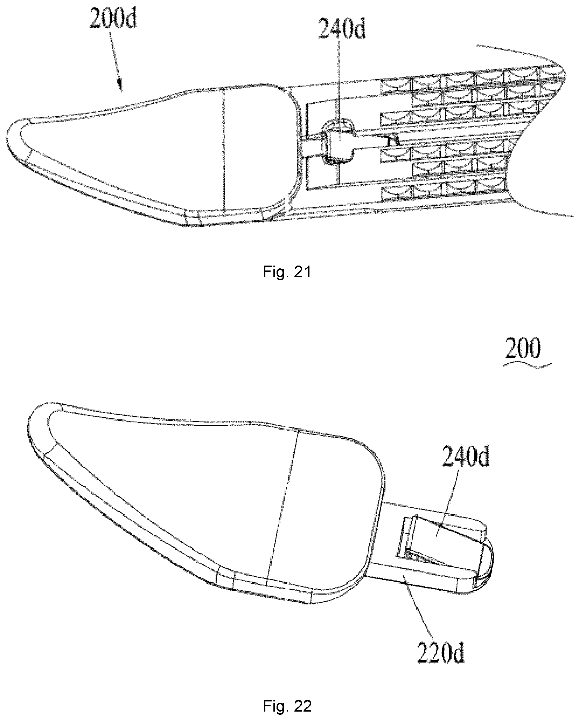

Fig. 21 to Fig. 22 show the separation member cooperatively used with the surgical instrument provided in a fifth implementation of the present invention.

A difference between the separation member 200d of this implementation and the separation member in the second implementation is mainly described below.

In the second implementation, the clipping portion itself has no elasticity, so that the support portion provides the elastic force for the clipping portion to cause the clipping portion to have a trend to be clipped in the clipping port of the surgical instrument.

In this implementation, the clipping portion 240d is at least partially made of the elastic material, so that the clipping portion 240d has a trend to be clipped in the clipping port of the surgical instrument. That is to say, other elastomers are not extra disposed to provide the elastic force for the clipping portion 240d, so that the structure is simpler.

In this implementation, the near end of the clipping portion 240d is connected to the mounting portion 220d of the separation member 200d. The clipping portion 240d is deformed. The far end of the clipping portion 240d rotates around the near end, so that the clipping portion 240d is clipped in the clipping port or detached from the clipping port, and finally, the separation member 200d is switched between the first state and the second state. It is to be noted that, extra rotating shaft is not disposed on the near end, and the far end rotates around the junction of the near end and the mounting portion 220d.

Therefore, it may be seen that, the separation member 200d in this implementation is simpler in structure and lower in cost.

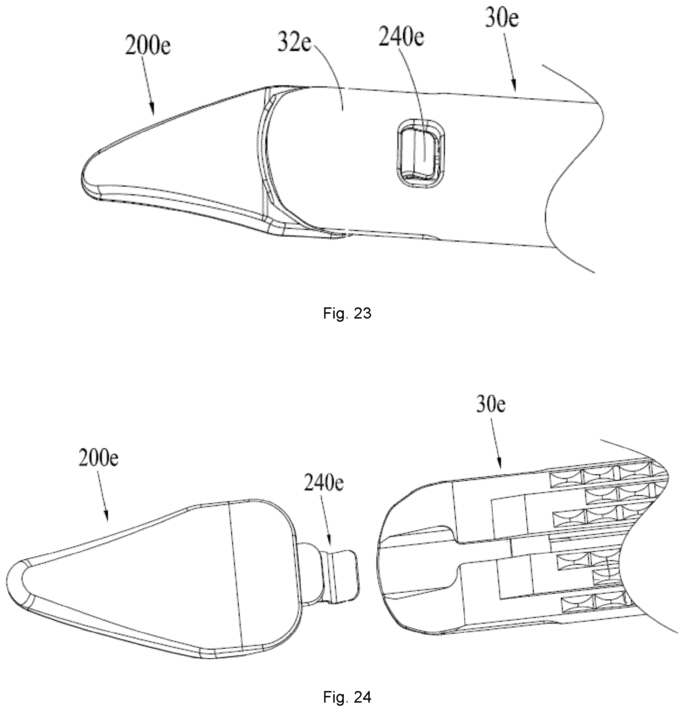

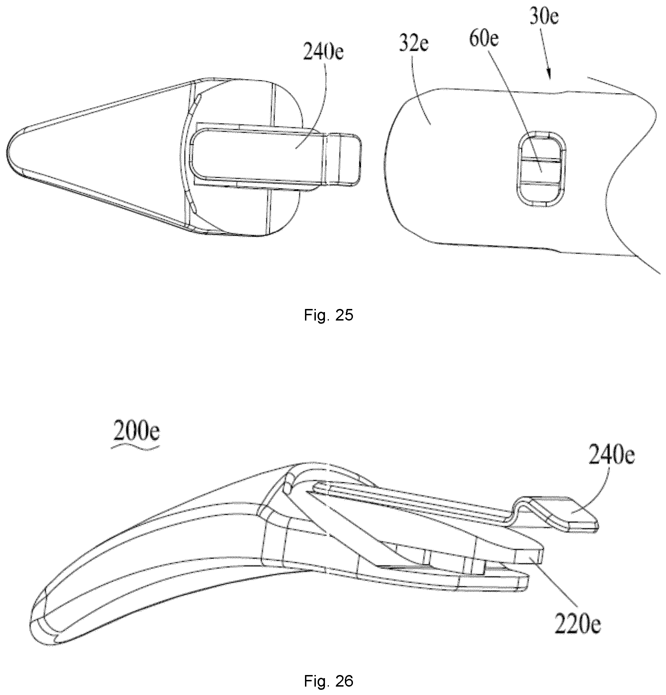

Fig. 23 to Fig. 26 show the separation member cooperatively used with the surgical instrument provided in a sixth implementation of the present invention.

A difference between the separation member 200e of this implementation and the separation member in the fifth implementation is mainly described below.

In the fifth implementation, the clipping portion is disposed on the front surface (second side surface) of the mounting portion of the separation member, and is mated with the clipping port disposed in the surgical instrument.

In this implementation, the clipping portion 240e is disposed on the back surface of the mounting portion 220e of the separation member 200e. The clipping port 60e is disposed on the back surface 32e of the staple abutting seat 30e. The clipping portion 240e disposed on the back surface of the mounting portion 220e is mated with the clipping port 60e disposed on the back surface 32e of the staple abutting seat 30e. Therefore, the separation member 200e can be detachably connected to the surgical instrument.

Since the clipping port 60e is disposed on the back surface 32e of the staple abutting seat 30e, the clipping port is not affected by the staple cartridge base. Therefore, the operating member of the packaging box may operate on the back surface 32e of the staple abutting seat 30e, so as to mount and dismount the separation member 200e. Therefore, the operating space is larger, and the operation is more convenient.

The far end of the clipping portion 240e is connected to the separation member 200e in the manner of welding. The clipping portion 240e itself has the elasticity, so that switching between the first state and the second state can be realized, and details are not described.

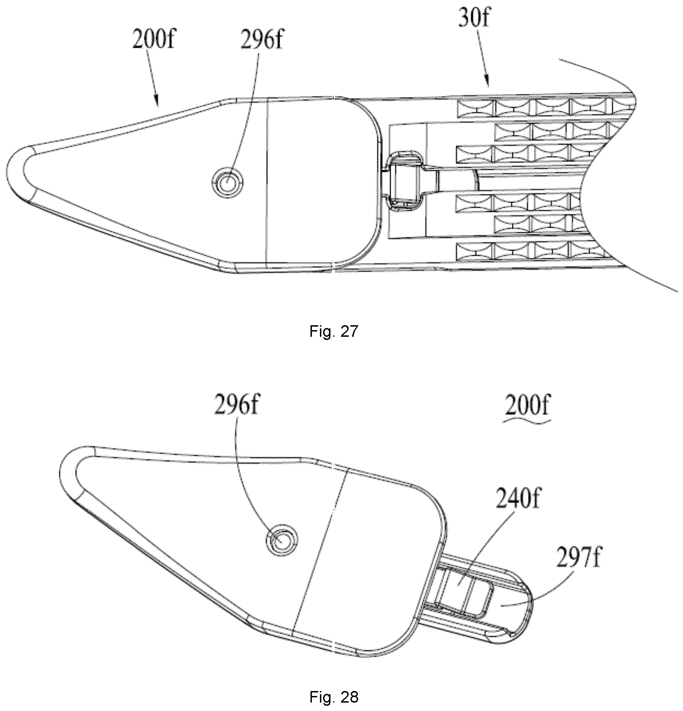

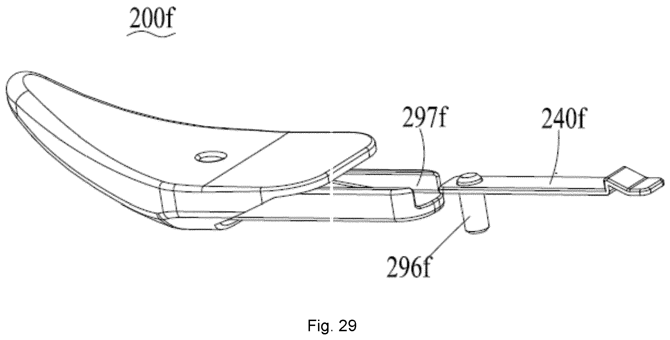

Fig. 27 to Fig. 29 show the separation member cooperatively used with the surgical instrument provided in a seventh implementation of the present invention.

A difference between the separation member 200f of this implementation and the separation member in the fifth implementation is mainly described below.

In the fifth implementation, the near end of the clipping portion is fixed to the mounting portion of the separation member, and the far end of the clipping portion is in mated connection with the clipping port of the surgical instrument.

In this implementation, the far end of the clipping portion 240f is fixed to the portion of the separation member 200f other than the clipping portion 240f, and the near end of the clipping portion 240f is in mated connection with the clipping port of the surgical instrument.

In this implementation, the separation member 200f is provided with an accommodation cavity 297f. The accommodation cavity 297f extends from the near end of the mounting portion in a direction toward the working portion. The accommodation cavity 297f is partially located in the working portion. A mounting shaft 296f vertically extending is disposed on the far end of the accommodation cavity 297f. The clipping portion 240f is provided with a mounting hole that can be sleeved on the mounting shaft 296f. The mounting hole is mated with the mounting shaft 296f to achieve the mated connection between the far end of the clipping portion 240f and the separation member 200f. The near end of the clipping portion 240f may rotate around the far end to achieve the clipping and detaching of the clipping port.

It is to be noted that, in this implementation, although the mounting shaft 296f is a cylindrical shaft and the mounting hole is a circular hole, the clipping portion 240f does not rotate around the axis of the mounting shaft 296f. The rotation axis of the clipping portion 240f is the same as that in the foregoing implementation. The rotation axis of the clipping portion 240f is parallel to a working surface of the separation member 200f.

To sum up, in the present invention, the doctor first uses the separation member to strip the target tissue from other tissue, and then moves the surgical instrument forward, to cause the target tissue to be between the staple cartridge base and the staple abutting seat, so as to operate the surgical instrument to cut and suture the target tissue. By using the separation member to strip the target tissue, the operation of the doctor can be easier and more convenient. In addition, by means of the clipping mechanism, the separation member is in elastically-mated connection with the surgical instrument, so that reliable connection and convenient dismounting can be realized.

In the present invention, the elastomer abuts against the end surface of the mounting end, so that the end surface of the far end of the mounting end may be prevented from scratching the tissue to the greatest extend, and the risk that the tissue is sandwiched in the gap between the end surface of the far end of the mounting end and the separation member may be reduced to the greatest extend.

In the present invention, the packaging box can not only provide packaging for the separation member, but also serve as a disassembling tool of the separation member relative to the surgical instrument, of which design is very artful. In the present invention, the elastomer may not only prevent the tissue from being scratched, but also prevent the separation member from accidentally falling off when the separation member is accommodated in the packaging box.

It should be understood that, although this specification is described in terms of implementations, not every implementation only includes an independent technical solution, and this description in the specification is only for clarity. Those skilled in the art should take the description as a whole, and the technical solutions in each implementation may also be appropriately combined to form other implementations that can be understood by those skilled in the art.

## Claims

1. A separation member for a surgical instrument, comprising a mounting portion (220) and a working portion (230), the mounting portion (220) is connected to the working portion (230), the mounting portion (220) comprises a clipping portion (240), the working portion (230) is configured to strip a tissue, **characterized in that** the clipping portion (240) is in elastically-mated connection with or detached from a surgical instrument (100), so that the mounting portion (220) is able to be detachably connected to the surgical instrument (100), the mounting portion (220) comprises an insertion portion (222), the clipping portion (240) has a first end (241) and a second end (242), the first end (241) is connected to the insertion portion (222), the second end (242) rotates around the first end (241), the second end (242) is in elastically-mated connection with or detached from the surgical instrument (100), the insertion portion (222) and the clipping portion (240) are configured to insert into an insertion channel (50) of the surgical instrument (100).

2. The separation member as claimed in claim 1, wherein
in a first state, the clipping portion (240) is clipped in a clipping port (60) of the surgical instrument (100) under an action of elastic force, so that the separation member (200) is in elastically-mated connection with the surgical instrument (100);
in a second state, the clipping portion (240) overcomes the elastic force to disengage from the clipping port (60), so that the separation member (200) is detached from the surgical instrument (100).

3. The separation member as claimed in claim 2, wherein the first end (241) comprises a rotating shaft (243), the insertion portion (222) is provided with a shaft hole (224), the rotating shaft (243) is mounted in the shaft hole (224), the rotating shaft (243) rotates in the shaft hole (224) to cause the clipping portion (240) to rotate.

4. The separation member as claimed in claim 2, wherein the first end (241) is mounted to a first side surface (229) of the insertion portion (222), a second side surface (227) of the insertion portion (222) is located on an opposite side of the first side surface (229), the insertion portion (222) is provided with an opening (228) through the first side surface (229) and the second side surface (227), so that the second end (242) protrudes from the first side surface (229) by extending through the opening (228) from the second side surface (227).

5. The separation member as claimed in claim 4, wherein the first side surface (229) is recessed inward to form accommodating space (226), the accommodating space (226) comprises the opening (228) penetrating the insertion portion (222), the second end (242) protrudes from the opening (228) to be in clipped connection with the clipping port (60) in the first state.

6. The separation member as claimed in claim 5, wherein the separation member (200) comprises a support portion (250), the support portion (250) elastically supports the clipping portion (240), when the separation member (200) is in the first state, a part of the clipping portion (240) is accommodated in the accommodating space (226), a part of the support portion (250) is also accommodated in the accommodating space (226).

7. The separation member as claimed in claim 2, wherein the separation member (200) comprises a support portion (250), the support portion (250) elastically supports the clipping portion (240), the support portion (250) has a first portion (251) and a second portion (252), the insertion portion (222) has a first side surface (229) and a second side surface (227) arranged opposite to each other, the first portion (251) is connected to the first side surface (229), the second portion (252) abuts against the clipping portion (240) and provides the elastic force for the clipping portion (240),

8. The separation member as claimed in claim 7, wherein the first end (241) comprises a rotating shaft (243), the insertion portion (222) is provided with a shaft hole (224), the shaft hole (224) is partially open, a part of the rotating shaft (243) is arranged on the shaft hole (224), the support portion (250) covers an open portion of the shaft hole (224).

9. The separation member as claimed in claim 7, wherein the separation member (200) comprises a second elastomer, the second elastomer is arranged between the clipping portion (240) and the support portion (250), the second elastomer and the support portion (250) provide the elastic force for the clipping portion (240) together.

10. The separation member as claimed in claim 2, wherein the second end (242) comprises a clipping surface (244) and a slope (246) disposed with the clipping surface (244) at an angle, the clipping surface (244) extends vertically, the clipping surface (244) abuts against a sidewall of the clipping port (60) to prevent the separation member (200) from falling off the surgical instrument (100), the slope (246) is configured to guide the second end (242) into the clipping port (60).

11. The separation member as claimed in any one of claims 1 to 10, wherein a second wedge portion (239) is disposed on a near end of the working portion (230), the second wedge portion (239) has a second slope (238), the separation member (200) is mounted to the staple abutting seat (30), a first slope (38) of a first wedge portion (39) at a far end of the staple abutting seat (30) abuts against the second slope (238)

12. The separation member as claimed in any one of claims 1 to 10, wherein the separation member (200) has an elastic member (280), the elastic member (280) is arranged on a surface of the working portion (230).

13. The separation member as claimed in claim 12, wherein the separation member (200) is provided with a slot (290), the elastic member (280) is disposed in the slot (290), an outside surface of the elastic member (280) protrudes from a second outer surface (202) of the separation member (200) or is flush with the second outer surface (202).

14. The separation member as claimed in claim 12, wherein the elastic member (280) is in a bag shape, at least of the working portion (230) is inserted in the elastic member (280).

15. The separation member as claimed in claim 2, wherein the first end (241) comprises a rotating shaft (234b), the first end of the clipping portion (240) is provided with a pin hole (299b) extending through the rotating shaft (234b), the mounting portion of the separation member (200) is provided with circular holes (298b) corresponding to two end portions of the pin hole (299b), the rotating shaft (234b) extends through a first circular hole (298b) of the circular holes (298b), the pin hole (299b) and a second circular hole (298b) of the circular holes (298b), so that the first end of the clipping portion (240) is rotatably mounted on the mounting portion (220).

16. The separation member as claimed in any one of claims 1 to 10, wherein the clipping portion (240) is at least partially made of the elastic material, a near end of the clipping portion (240) is connected to the mounting portion (220), the clipping portion (240) is deformed, a far end of the clipping portion (240) rotates around the near end, so that the clipping portion (240) is clipped in a clipping port (60) or detached from a clipping port (60) of the surgical instrument (100).

17. An surgical instrument, comprising an end effector (18), the end effector (18) is connected to the separation member (200) as claimed in any one of claims 1 to 16, the separation member (200) is detachably connected to the end effector (18) of the surgical instrument (100).

## Patentansprüche

1. Ein Trennelement für ein chirurgisches Instrument, umfassend einen Befestigungsabschnitt (220) und einen Arbeitsabschnitt (230), wobei der Befestigungsabschnitt (220) mit dem Arbeitsabschnitt (230) verbunden ist, der Befestigungsabschnitt (220) einen Klemmabschnitt (240) umfasst, der Arbeitsabschnitt (230) konfiguriert ist, ein Gewebe abzustreifen, **dadurch gekennzeichnet, dass** der Klemmabschnitt (240) in elastisch ineinandergreifender Verbindung mit einem chirurgischen Instrument (100) steht oder von diesem gelöst ist, so dass der Befestigungsabschnitt (220) lösbar mit dem chirurgischen Instrument (100) verbunden sein kann, der Befestigungsabschnitt (220) einen Einführabschnitt (222) umfasst, der Klemmabschnitt (240) ein erstes Ende (241) und ein zweites Ende (242) aufweist, das erste Ende (241) mit dem Einführabschnitt (222) verbunden ist, das zweite Ende (242) um das erste Ende (241) dreht, das zweite Ende (242) in elastisch ineinandergreifender Verbindung mit dem chirurgischen Instrument (100) steht oder von diesem gelöst ist, der Einführabschnitt (222) und der Klemmabschnitt (240) konfiguriert sind, in einen Einführkanal (50) des chirurgischen Instruments (100) eingeführt zu werden.

2. Das Trennelement nach Anspruch 1, wobei
in einem ersten Zustand der Klemmabschnitt (240) unter Einwirkung einer elastischen Kraft in einen Klemmanschluss (60) des chirurgischen Instruments (100) eingeklemmt wird, so dass das Trennelement (200) in einer elastisch ineinandergreifenden Verbindung mit dem chirurgischen Instrument (100) steht;
in einem zweiten Zustand der Klemmabschnitt (240) die elastische Kraft überwindet, um sich aus dem Klemmanschluss (60) auszuklinken, so dass das Trennelement (200) vom chirurgischen Instrument (100) gelöst ist.

3. Das Trennelement nach Anspruch 2, wobei das erste Ende (241) eine Drehwelle (243) umfasst, der Einführabschnitt (222) mit einer Wellenbohrung (224) versehen ist, die Drehwelle (243) in der Wellenbohrung (224) gelagert ist, die Drehwelle (243) sich in der Wellenbohrung (224) dreht, um den Klemmabschnitt (240) zu veranlassen sich zu drehen.

4. Das Trennelement nach Anspruch 2, wobei das erste Ende (241) an einer ersten Seitenfläche (229) des Einführabschnitts (222) angebracht ist, wobei sich eine zweite Seitenfläche (227) des Einführabschnitts (222) auf einer der ersten Seitenfläche (229) gegenüberliegenden Seite befindet, der Einführabschnitt (222) mit einer Öffnung (228) durch die erste Seitenfläche (229) und die zweite Seitenfläche (227) versehen ist, so dass das zweite Ende (242) aus der ersten Seitenfläche (229) herausragt, indem es sich durch die Öffnung (228) von der zweiten Seitenfläche (227) aus erstreckt.

5. Das Trennelement nach Anspruch 4, wobei die erste Seitenfläche (229) nach innen vertieft ist, um einen Aufnahmeraum (226) zu bilden, wobei der Aufnahmeraum (226) die Öffnung (228) umfasst, die den Einführabschnitt (222) durchdringt, wobei das zweite Ende (242) aus der Öffnung (228) herausragt, um in dem ersten Zustand in einer geklemmten Verbindung mit dem Klemmanschluss (60) zu stehen.

6. Das Trennelement nach Anspruch 5, wobei das Trennelement (200) einen Stützabschnitt (250) umfasst, wobei der Stützabschnitt (250) den Klemmabschnitt (240) elastisch stützt, wenn sich das Trennelement (200) in dem ersten Zustand befindet, wobei ein Teil des Klemmabschnitts (240) in dem Aufnahmeraum (226) aufgenommen ist, und ein Teil des Stützabschnitts (250) ebenfalls in dem Aufnahmeraum (226) aufgenommen ist.

7. Das Trennelement nach Anspruch 2, wobei das Trennelement (200) einen Stützabschnitt (250) umfasst, wobei der Stützabschnitt (250) den Klemmabschnitt (240) elastisch stützt, der Stützabschnitt (250) einen ersten Abschnitt (251) und einen zweiten Abschnitt (252) aufweist, der Einführabschnitt (222) eine erste Seitenfläche (229) und eine zweite Seitenfläche (227) aufweist, die einander gegenüberliegend angeordnet sind, der erste Abschnitt (251) mit der ersten Seitenfläche (229) verbunden ist, der zweite Abschnitt (252) an dem Klemmabschnitt (240) anliegt und die elastische Kraft für den Klemmabschnitt (240) bereitstellt.

8. Das Trennelement nach Anspruch 7, wobei das erste Ende (241) eine Drehwelle (243) umfasst, der Einführabschnitt (222) mit einer Wellenbohrung (224) versehen ist, die Wellenbohrung (224) teilweise offen ist, ein Teil der Drehwelle (243) an der Wellenbohrung (224) angeordnet ist, der Stützabschnitt (250) einen offenen Abschnitt der Wellenbohrung (224) abdeckt.

9. Das Trennelement nach Anspruch 7, wobei das Trennelement (200) ein zweites Elastomer umfasst, das zweite Elastomer zwischen dem Klemmabschnitt (240) und dem Stützabschnitt (250) angeordnet ist, das zweite Elastomer und der Stützabschnitt (250) gemeinsam die elastische Kraft für den Klemmabschnitt (240) bereitstellen.

10. Das Trennelement nach Anspruch 2, wobei das zweite Ende (242) eine Klemmfläche (244) und eine Schräge (246) umfasst, die in einem Winkel zur Klemmfläche (244) angeordnet ist, wobei sich die Klemmfläche (244) vertikal erstreckt, die Klemmfläche (244) an einer Seitenwand des Klemmanschlusses (60) anliegt, um zu verhindern, dass das Trennelement (200) von dem chirurgischen Instrument (100) abfällt, wobei die Schräge (246) konfiguriert ist, das zweite Ende (242) in den Klemmanschluss (60) zu führen.

11. Das Trennelement nach einem der Ansprüche 1 bis 10, wobei ein zweiter Keilabschnitt (239) an einem nahen Ende des Arbeitsabschnitts (230) angeordnet ist, der zweite Keilabschnitt (239) eine zweite Schräge (238) aufweist, das Trennelement (200) an dem Klammeranschlagsitz (30) angebracht ist, eine erste Schräge (38) eines ersten Keilabschnitts (39) an einem entfernten Ende des Klammeranschlagsitzes (30) an der zweiten Schräge (238) anliegt.

12. Das Trennelement nach einem der Ansprüche 1 bis 10, wobei das Trennelement (200) ein elastisches Element (280) aufweist, wobei das elastische Element (280) auf einer Oberfläche des Arbeitsabschnitts (230) angeordnet ist.

13. Das Trennelement nach Anspruch 12, wobei das Trennelement (200) mit einem Schlitz (290) versehen ist, das elastische Element (280) in dem Schlitz (290) angeordnet ist, eine Außenfläche des elastischen Elements (280) aus einer zweiten Außenfläche (202) des Trennelements (200) hervorragt oder bündig mit der zweiten Außenfläche (202) abschließt.

14. Das Trennelement nach Anspruch 12, wobei das elastische Element (280) eine Beutelform aufweist, wobei zumindest von dem Arbeitsabschnitt (230) in das elastische Element (280) eingeführt ist.

15. Das Trennelement nach Anspruch 2, wobei das erste Ende (241) eine Drehwelle (234b) umfasst, das erste Ende des Klemmabschnitts (240) mit einem Stiftloch (299b) versehen ist, das sich durch die Drehwelle (234b) erstreckt, und der Befestigungsabschnitt des Trennelements (200) mit kreisförmigen Löchern (298b) versehen ist, die zu zwei Endabschnitten des Stiftlochs (299b) korrespondieren, die Drehwelle (234b) sich durch ein erstes kreisförmiges Loch (298b) der kreisförmigen Löcher (298b), das Stiftloch (299b) und ein zweites kreisförmiges Loch (298b) der kreisförmigen Löcher (298b) hindurch erstreckt, so dass das erste Ende des Klemmabschnitts (240) drehbar an dem Befestigungsabschnitt (220) angebracht ist.

16. Das Trennelement nach einem der Ansprüche 1 bis 10, wobei der Klemmabschnitt (240) zumindest teilweise aus dem elastischen Material hergestellt ist, ein nahes Ende des Klemmabschnitts (240) mit dem Befestigungsabschnitt (220) verbunden ist, der Klemmabschnitt (240) verformt ist, ein entferntes Ende des Klemmabschnitts (240) sich um das nahe Ende dreht, so dass der Klemmabschnitt (240) in einen Klemmanschluss (60) des chirurgischen Instruments (100) eingeklemmt oder von einem Klemmanschluss (60) des chirurgischen Instruments (100) gelöst ist.

17. Ein chirurgisches Instrument, umfassend einen Endeffektor (18), wobei der Endeffektor (18) mit dem Trennelement (200) nach einem der Ansprüche 1 bis 16 verbunden ist, wobei das Trennelement (200) lösbar mit dem Endeffektor (18) des chirurgischen Instruments (100) verbunden ist.

## Revendications

1. Organe de séparation pour un instrument chirurgical, comprenant une portion de montage (220) et une portion de travail (230), la portion de montage (220) est reliée à la portion de travail (230), la portion de montage (220) comprend une portion de clipsage (240), la portion de travail (230) est configurée pour dénuder un tissu, **caractérisé en ce que** la portion de clipsage (240) est en liaison de contact élastique avec un instrument chirurgical (100) ou détachée de ce dernier, de sorte que la portion de montage (220) peut être reliée de manière détachable à l'instrument chirurgical (100), la portion de montage (220) comprend une portion d'insertion (222), la portion de clipsage (240) présente une première extrémité (241) et une seconde extrémité (242), la première extrémité (241) est reliée à la portion d'insertion (222), la seconde extrémité (242) tourne autour de la première extrémité (241), la seconde extrémité (242) est en liaison de contact élastique avec l'instrument chirurgical (100) ou détachée de ce dernier, la portion d'insertion (222) et la portion de clipsage (240) sont configurées pour s'insérer dans un canal d'insertion (50) de l'instrument chirurgical (100).

2. Organe de séparation selon la revendication 1, dans lequel
dans un premier état, la portion de clipsage (240) est clipsée dans un orifice de clipsage (60) de l'instrument chirurgical (100) sous l'effet d'une force élastique, de sorte que l'organe de séparation (200) est en liaison de contact élastique avec l'instrument chirurgical (100) ;
dans un second état, la portion de clipsage (240) surmonte la force élastique pour se désengager de l'orifice de clipsage (60), de sorte que l'organe de séparation (200) est détaché de l'instrument chirurgical (100).

3. Organe de séparation selon la revendication 2, dans lequel la première extrémité (241) comprend un arbre rotatif (243), la portion d'insertion (222) est dotée d'un trou pour arbre (224), l'arbre rotatif (243) est monté dans le trou pour arbre (224), l'arbre rotatif (243) tourne dans le trou pour arbre (224) pour faire tourner la portion de clipsage (240).

4. Organe de séparation selon la revendication 2, dans lequel la première extrémité (241) est montée sur une première surface latérale (229) de la portion d'insertion (222), une seconde surface latérale (227) de la portion d'insertion (222) est située sur un côté opposé de la première surface latérale (229), la portion d'insertion (222) est dotée d'une ouverture (228) traversant la première surface latérale (229) et la seconde surface latérale (227), de sorte que la seconde extrémité (242) fait saillie de la première surface latérale (229) en s'étendant à travers l'ouverture (228) depuis la seconde surface latérale (227).

5. Organe de séparation selon la revendication 4, dans lequel la première surface latérale (229) est en retrait vers l'intérieur pour former un espace de logement (226), l'espace de logement (226) comprend l'ouverture (228) pénétrant la portion d'insertion (222), la seconde extrémité (242) fait saillie de l'ouverture (228) pour être en liaison clipsée avec l'orifice de clipsage (60) dans le premier état.

6. Organe de séparation selon la revendication 5, dans lequel l'organe de séparation (200) comprend une portion de support (250), la portion de support (250) supporte élastiquement la portion de clipsage (240), lorsque l'organe de séparation (200) est dans le premier état, une partie de la portion de clipsage (240) est logée dans l'espace de logement (226), une partie de la portion de support (250) est également logée dans l'espace de logement (226).

7. Organe de séparation selon la revendication 2, dans lequel l'organe de séparation (200) comprend une portion de support (250), la portion de support (250) supporte élastiquement la portion de clipsage (240), la portion de support (250) présente une première portion (251) et une seconde portion (252), la portion d'insertion (222) présente une première surface latérale (229) et une seconde surface latérale (227) agencées l'une en face de l'autre, la première portion (251) est reliée à la première surface latérale (229), la seconde portion (252) est en butée contre la portion de clipsage (240) et fournit la force élastique nécessaire à la portion de clipsage (240).

8. Organe de séparation selon la revendication 7, dans lequel la première extrémité (241) comprend un arbre rotatif (243), la portion d'insertion (222) est dotée d'un trou pour arbre (224), le trou pour arbre (224) est partiellement ouvert, une partie de l'arbre rotatif (243) est agencée sur le trou pour arbre (224), la portion de support (250) recouvre une portion ouverte du trou pour arbre (224).

9. Organe de séparation selon la revendication 7, dans lequel l'organe de séparation (200) comprend un second élastomère, le second élastomère est agencé entre la portion de clipsage (240) et la portion de support (250), le second élastomère et la portion de support (250) fournissent ensemble la force élastique pour la portion de clipsage (240).

10. Organe de séparation selon la revendication 2, dans lequel la seconde extrémité (242) comprend une surface de clipsage (244) et une pente (246) disposée avec la surface de clipsage (244) à un angle, la surface de clipsage (244) s'étend verticalement, la surface de clipsage (244) est en butée contre une paroi latérale de l'orifice de clipsage (60) pour empêcher l'organe de séparation (200) de tomber de l'instrument chirurgical (100), la pente (246) est configurée pour guider la seconde extrémité (242) dans l'orifice de clipsage (60).

11. Organe de séparation selon l'une des revendications 1 à 10, dans lequel une seconde portion de coin (239) est disposée sur une extrémité proche de la portion de travail (230), la seconde portion de coin (239) présente une seconde pente (238), l'organe de séparation (200) est monté sur le siège de butée d'agrafe (30), une première pente (38) d'une première portion de coin (39) à une extrémité éloignée du siège de butée d'agrafe (30) est en butée contre la seconde pente (238).

12. Organe de séparation selon l'une des revendications 1 à 10, dans lequel l'organe de séparation (200) présente un organe élastique (280), l'organe élastique (280) est agencé sur une surface de la portion de travail (230).

13. Organe de séparation selon la revendication 12, dans lequel l'organe de séparation (200) est doté d'une fente (290), l'organe élastique (280) est disposé dans la fente (290), une surface extérieure de l'organe élastique (280) fait saillie d'une seconde surface extérieure (202) de l'organe de séparation (200) ou est à fleur de la seconde surface extérieure (202).

14. Organe de séparation selon la revendication 12, dans lequel l'organe élastique (280) est en forme de sac, au moins de la portion de travail (230) est insérée dans l'organe élastique (280).

15. Organe de séparation selon la revendication 2, dans lequel la première extrémité (241) comprend un arbre rotatif (234b), la première extrémité de la portion de clipsage (240) est dotée d'un trou de goupille (299b) s'étendant à travers l'arbre rotatif (234b), la portion de montage de l'organe de séparation (200) est dotée de trous circulaires (298b) correspondant à deux portions d'extrémité du trou de goupille (299b), l'arbre rotatif (234b) s'étend à travers un premier trou circulaire (298b) des trous circulaires (298b), le trou de goupille (299b) et un second trou circulaire (298b) des trous circulaires (298b), de sorte que la première extrémité de la portion de clipsage (240) soit montée en rotation sur la portion de montage (220).

16. Organe de séparation selon l'une des revendications 1 à 10, dans lequel la portion de clipsage (240) est au moins partiellement réalisée en matériau élastique, une extrémité proche de la portion de clipsage (240) est reliée à la portion de montage (220), la portion de clipsage (240) est déformée, une extrémité éloignée de la portion de clipsage (240) tourne autour de l'extrémité proche, de sorte que la portion de clipsage (240) est clipsée dans un orifice de clipsage (60) ou détachée d'un orifice de clipsage (60) de l'instrument chirurgical (100).

17. Instrument chirurgical, comprenant un effecteur terminal (18), l'effecteur terminal (18) est relié à l'organe de séparation (200) selon l'une des revendications 1 à 16, l'organe de séparation (200) est relié de manière détachable à l'effecteur terminal (18) de l'instrument chirurgical (100).
